# EUROPEAN PATENT SPECIFICATION

(11) **EP 0 712 853 B1**
(45) Date of publication and mention of the grant of the patent: **28.02.2001**
(21) Application number: 94921846.5
(22) Date of filing: 01.08.1994
(51) Int. Cl.: C07D 409/06, C07D 335/06, A01N 43/56, A01N 43/18

(54) **PYRAZOLE DERIVATIVE**
PYRAZOL-DERIVATE
DERIVE DE PYRAZOLE

(30) Priority: 02.08.1993 JP 19142893; 11.04.1994 JP 7178894
(43) Date of publication of application: 22.05.1996
(73) Proprietor: IDEMITSU KOSAN COMPANY LIMITED, Tokyo 100-0005 (JP)
(72) Inventor: NAKAMURA, Kazufumi Idemitsu Kosan Co., Ltd., Chiba-ken 299-02 (JP); KOIKE, Kazuyoshi Idemitsu Kosan Co. Ltd., Chiba-ken 299-02 (JP); SAKAMOTO, Masashi Idemitsu Kosan Co., Ltd., Chiba-ken 299-02 (JP); NASUNO, Ichiro Idemitsu Kosan Co., Ltd., Chiba-ken 299-02 (JP)
(74) Representative: Gille Hrabal Struck Neidlein Prop Roos
(86) International application number: JP9401264
(87) International publication number: WO9504054

(56) References cited:
- JP-A- 2 000 173
- JP-A- 4 257 503
- JP-A- 63 122 672
- JP-A- 63 122 673
- JP-A- 63 170 365
- US-A- 5 015 658
- CHEMICAL ABSTRACTS, vol. 107, no. 5, 3 August 1987 Columbus, Ohio, US; abstract no. 39613e, K. SHUTO: "Preparation of benzopyran and benzothiopyran derivatives as antitumor agents." page 681; column 1; XP002016247 & JP-A-62 053 981 9 March 1987
- CHEMICAL ABSTRACTS, vol. 124, no. 19, 6 May 1996 Columbus, Ohio, US; abstract no. 261028c, M. SHIBATA ET AL.: "Preparation of pyrazole derivatives as herbicidal compositions." page 1268; column 2; XP002016248 & WO-A-96 00008 (IDEMITSU KOSAN CO., LTD.) 4 January 1996
- CHEMICAL ABSTRACTS, vol. 124, no. 25, 17 June 1996 Columbus, Ohio, US; abstract no. 343114s, K. NAKAMURA ET AL.: "Preparation of thiochroman derivatives as herbicides." page 1310; column 2; XP002016249 & JP-A-08 026 914 (IDEMITSU KOSAN CO., LTD.) 30 January 1996

## Description

### Technical Field

The present invention relates to pyrazole derivatives, herbicides containing them as active ingredients, and intermediate compounds suitable for the production of the above pyrazole derivatives.

### Technical Background and Problems to be Solved by the Invention

Herbicides are very important chemicals for the labor-saving in weed control work and for increasing the productivity for agricultural and horticultural products. Herbicides have been therefore actively studied and developed for many years, and a variety of chemicals are practically used at present. Even today, however, it is desired to develop novel chemicals having distinguished herbicidal properties, particularly chemicals which are free of phytoxicity to cultured crops and can control target weeds selectively in a low dosage.

During a growing period of corn, etc., a triazine-based herbicide such as atrazine and acid anilide-based herbicides such as alachlor and metolachlor have been conventionally used. However, atrazine shows low efficacy to gramineous weeds, and alachlor and metolachlor show low efficacy to broad-leaved weeds. It is therefore difficult at present to control gramineous weeds and broad-leaved weeds together with a single herbicide. Further, the above herbicides are undesirable in view of an environmental problem due to their high dosage requirement.

Further, it is known that a paddy field grows not only paddy rice but also various weeds including annual gramineous weeds such as barnyard grass, annual cyperaceous weeds such as umbrella plant, annual broad-leaved weeds such as monochoria and toothcup and perennial weeds such as Sagittaria pygmaea miquel, largeleaf pondweed, oriental waterplantain, bulrush, needle-upright clubrush, Cyperus serotinus rottboell, water chestnut, arrowhead and dropwort. It is very important for rice cultivation to effectively control these weeds without causing phytotoxicity to paddy rice and by spraying a chemical in a small dosage in view of environmental pollution. It is known that chemicals having high herbicidal activity to barnyard grass are generally liable to cause phytotoxicity to paddy rice; and it is an especially essential object remaining to achieve to develop a chemical which exhibits high herbicidal activity to barnyard grass which is a gramineous weed, and has excellent intergenus selectivity between paddy rice and barnyard grass.

Meanwhile, it is already known that specific 4-benzoylpyrazole derivatives have herbicidal activity (see JP-A-63-122672, JP-A-63-122673, JP-A-63-170365, JP-A-1-52759, JP-A-2-173 and JP-A-2-288866). As a commercially available herbicide at present, there is a pyrazolate of the following chemical formula.

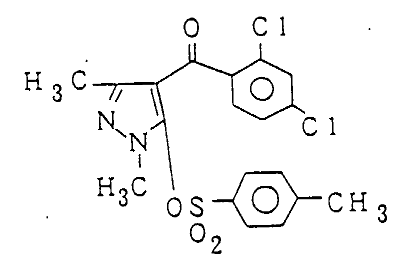

Further, the typical example (A) of the 4-benzoylpyrazole derivatives disclosed in the above publications (Compound No. 35 in JP-A-2-173) has the following chemical formula.

### Compound (A): Compound No. 35 described in JP-A-2-173

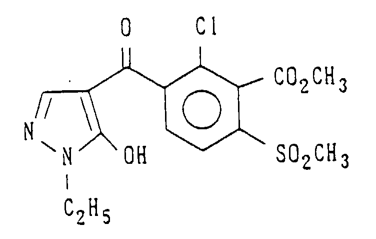

However, these 4-benzoylpyrazole derivatives have herbicidal activity, hut are insufficient in practical use. In particular, they are very inferior in herbicidal activity to gramineous weeds such as barnyard grass and green foxtail. Further, when used as a herbicide in a paddy field, the above chemicals may cause phytotoxicity to paddy rice since they have poor selectivity between paddy rice and gramineous weeds.

The present inventors have therefore proposed pyrazole derivatives having a thiochroman ring by already applying a patent (Japanese Patent Application No. 4-185526 and International Patent Publication W093/18031). Typical examples (B) and (C) of compounds disclosed in the specifications of the above prior applications are as follows.

### Compound (B): Compound No. 66 described in International Patent Publication WO93/18031

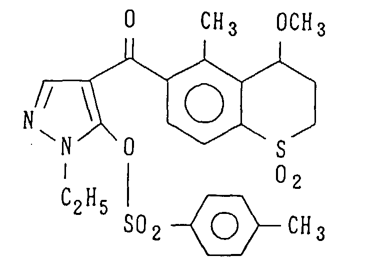

### Compound (C): Compound No. b-3 described in Japanese Patent Application No. 4-185526

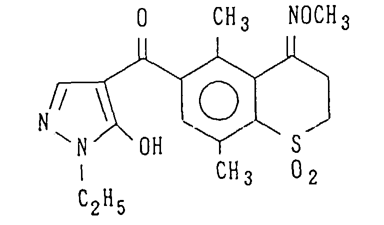

However, the above compounds have high herbicidal activity, while they still have room for improvement in safety to paddy rice.

The present invention has been made in view of the above circumstances, and the object thereof is to provide a pyrazole derivative which is free of phytotoxicity to corn and paddy rice and can control a wide range of upland field weeds and paddy field weeds, particularly barnyard grass in a paddy field, in a low dosage, a herbicide containing the pyrazole derivative, and further, an intermediate for producing the pyrazole derivative.

### Disclosure of the Invention

The first gist of the present invention consists in a pyrazole derivative of the general formula (I), 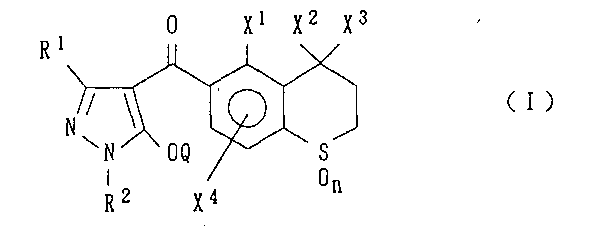 {wherein:
R¹ is a hydrogen atom or a C₁-C₄ alkyl group; R² is a C₁-C₄ alkyl group;
X¹ is a hydrogen atom, a halogen atom or a C₁-C₄ alkyl group;
each of X² and X³ is independently a C₁-C₄ alkyl group;
X⁴ is a hydrogen atom, or a halogen atom or C₁-C₄ alkyl group substituted on the 7-position or 8-position of a thiochroman ring;
Q is a hydrogen atom or a group of the formula of -A-B [in which:
A is -SO₂-, -CO- or -CH₂CO-; and
B is a C₁-C₈ alkyl group, a C₃-C₈ cycloalkyl group or a group of the formula (V), 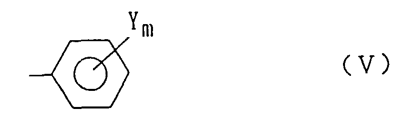 (in which:
Y is a halogen atom, a nitro group, a C₁-C₄ alkyl group, a C₁-C₄ alkoxy group or a halo C₁-C₄ alkyl group; and
m is an integer of 0, 1 or 2)]; and
n is an integer of 0, 1 or 2},
or a salt thereof.

Further, the second gist of the present invention consists in a herbicide containing the pyrazole derivative of the formula (I) and/or a salt thereof as an active ingredient.

Further, the third gist of the present invention consists in an aromatic carboxylic acid derivative which is useful for the production of the above pyrazole derivative of the general formula (I) and represented by the formula (II), 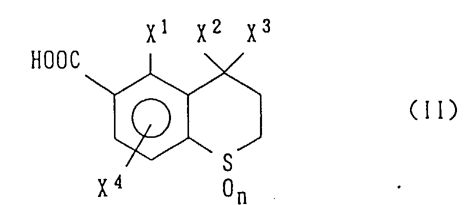 (wherein:
X¹ is a C₁-C₄ alkyl group or a halogen atom;
each of X² and X³ is independently a C₁-C₄ alkyl group; and
X⁴ is a hydrogen atom, or a C₁-C₄ alkyl group or halogen atom substituted on the 7-position or 8-position of a thiochroman ring),
or a salt thereof.

### Preferred Embodiments for Working the Invention

The pyrazole derivative of the present invention is a compound of the formula (I).

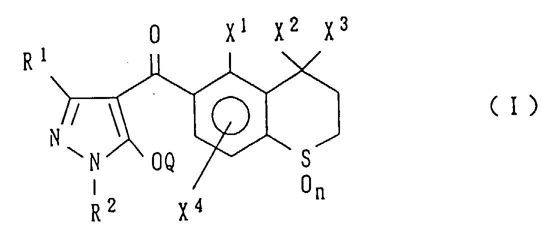

In the formula (I), R¹ is a hydrogen atom or a C₁-C₄ alkyl group, and R² is a C₁-C₄ alkyl group. The C₁-C₄ alkyl group for R¹ and R² includes methyl, ethyl, propyl such as n-propyl and i-propyl, and butyl such as n-butyl and i-butyl.

X¹ is a hydrogen atom, a halogen atom or a C₁-C₄ alkyl group. The halogen atom includes fluorine, chlorine, bromine and iodine. The C₁-C₄ alkyl group includes those specified concerning R¹ and R². X¹ is preferably a C₁-C₄ alkyl group, more preferably methyl.

Each of X² and X³ is independently a C₁-C₄ alkyl group. The C₁-C₄ alkyl group includes those specified concerning R¹ and R².

X⁴ is a hydrogen atom, a halogen atom or a C₁-C₄ alkyl group. The halogen atom includes those specified concerning X¹. The C₁-C₄ alkyl group includes those specified concerning R¹ and R². X⁴ is preferably a C₁-C₄ alkyl group, more preferably methyl. The substitution position is the 7-position or 8-position of the thiochroman ring, preferably the 8-position.

Q is a hydrogen atom or a group of -A-B, A is -SO₂-, -CO- or -CH₂CO-, and B is a C₁-C₈ alkyl group, C₃-C₈ cycloalkyl group or a group of the formula (V).

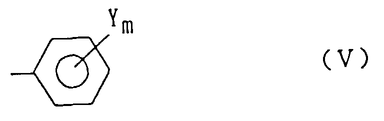

The C₁-C₈ alkyl group as one member for B includes methyl, ethyl, propyl, butyl, pentyl, hexyl, heptyl and octyl, and the alkyl group having at least 3 carbon atoms may be linear or branched. The C₃-C₈ cycloalkyl group as another member for B includes cyclopropyl, cyclobutyl, cyclopentyl, cyclohexyl, cycloheptyl and cyclooctyl.

In the group of the formula (V) as further another member for B, Y is a halogen atom, a nitro group, a C₁-C₄ alkyl group, a C₁-C₄ alkoxy group or a halo C₁-C₄ alkyl group. The halogen atom includes those specified concerning X¹ and X⁴. The C₁-C₄ alkyl group includes those specified concerning R¹ and R². The C₁-C₄ alkoxy group includes methoxy, ethoxy, propoxy optionally having a branch or butoxy optionally having a branch. The halo C₁-C₄ alkyl group includes those in which hydrogen atom(s) of the C₁-C₄ alkyl group specified concerning R¹ and R² is(are) replaced with halogen atom(s) specified concerning X¹ and X⁴. Specifically, the halo C₁-C₄ alkyl group includes -CH₂F, -CHF₂, -CF₃, -CF₂CF₃, - CCl₃ and -CH₂CF₃.

m showing the number of Y is an integer of 0, 1 or 2.

One preferred combination of A and B in the group of -A-B is a combination of A which is -SO₂- and B which is a C₁-C₈ alkyl group or a phenyl group substituted with 1 or 2 halogen atom(s), nitro group(s), C₁-C₄ alkyl group(s) or C₁-C₄ alkoxy group(s) (group of the formula (V) in which Y is halogen atom(s), nitro group(s), C₁-C₄ alkyl group(s) or C₁-C₄ alkoxy group(s) and m is 1 or 2). Another preferred combination of A and B is a combination of A which is -CO- or -CH₂CO- and B which is a C₁-C₈ alkyl or a halogen-substituted or unsubstituted phenyl (group of the formula (V) in which Y is halogen atom(s) and m is 0, 1 or 2).

n shows the number of oxygen, and is an integer of 0, 1 or 2. That is, when n is 0, a sulfide is represented. When n is 1, a sulfoxide is represented. When n is 2, a sulfone is represented.

Preferred pyrazole derivatives according to general formula (I) contain R¹ being a hydrogen atom.

Further preferred pyrazole derivatives according to general formula (I) contain R² being methyl or ethyl.

Yet another group of preferred pyrazole derivatives according to general formula (I) contains X¹ being a hydrogen atom, methyl or a chlorine atom.

Another group of preferred pyrazole derivatives according to general formula (I) contains X² and X³ being methyl.

A further preferred group of pyrazole derivatives according to general formula (I) contains X⁴ being a hydrogenation, or methyl or a chlorine atom substituted in position 8 of a thiochroman ring.

The pyrazole derivative of the general formula (I) in which Q is hydrogen, i.e., 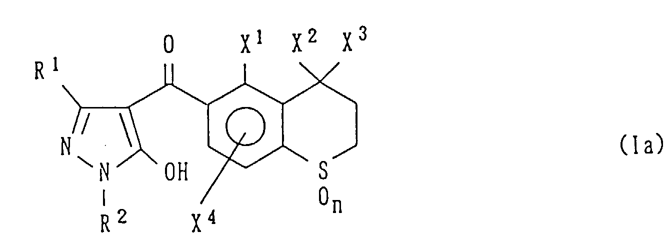 can have the following three structures due to tautomerism, and the pyrazole derivative of the present invention includes all of these structures.

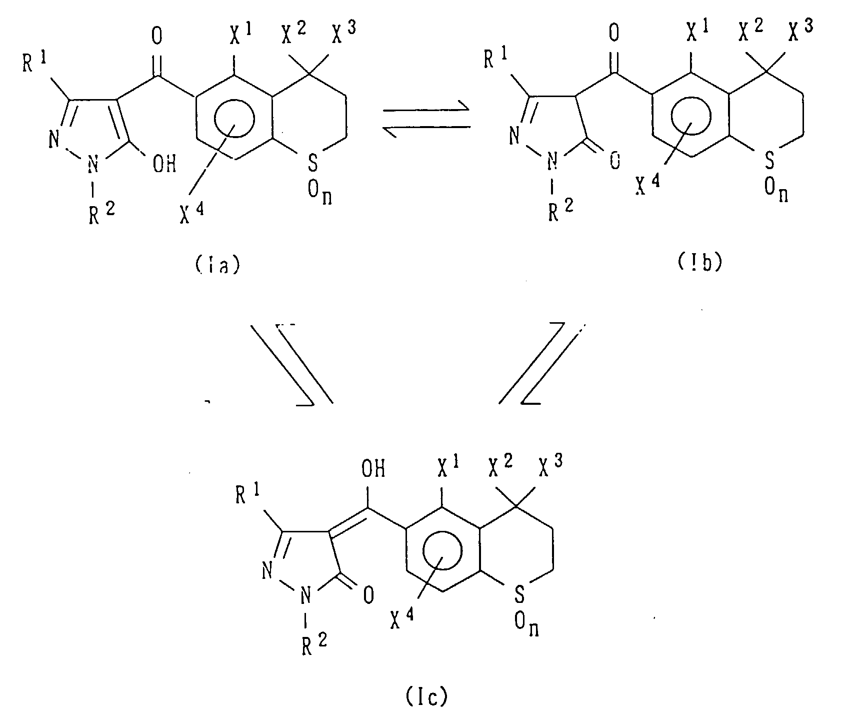

Further, the pyrazole derivative of the formula (Ia) is an acidic substance, and can therefore be easily converted to a salt by treating it with a base. This salt is also included in the pyrazole derivative of the present invention. The above base is not specially limited if it is known. For example, it is selected from organic bases such as amines and anilines and inorganic bases such as sodium compounds and potassium compounds. The amines include a monoalkylamine, a dialkylamine and a trialkylamine. The alkyl group of the alkylamines is generally a C₁-C₄ alkyl group. The anilines include aniline, a monoalkylaniline and a dialkylaniline. The alkyl group of the alkylanilines is generally a C₁-C₄ alkyl group. The sodium compounds include sodium hydroxide and sodium carbonate. The potassium compounds include potassium hydroxide and potassium carbonate.

The herbicide of the present invention contains the pyrazole derivative of the formula (I) and/or a salt thereof, provided by the present invention, as an active ingredient. These compounds are used by mixing them with a liquid carrier such as a solvent or a solid carrier such as a mineral fine powder and preparing the resultant mixtures in the form of a wettable powder, an emulsifiable concentrate, a dust or granules.' For imparting these compounds with emulsifiability, dispersibility or spreadability when the above preparations are formed, a surfactant may be added.

When the herbicide of the present invention is used in the form of a wettable powder, generally, 10 to 55 % by weight of the pyrazole derivative and/or its salt of the present invention, 40 to 88 % by weight of a solid carrier and 2 to 5 % by weight of a surfactant are mixed to prepare a composition, and the composition can be used. When the herbicide of the present invention is used in the form of an emulsifiable concentrate, generally, the emulsifiable concentrate can be prepared by mixing 20 to 50 % by weight of the pyrazole derivative and/or its salt of the present invention, 35 to 75 % by weight of a solvent and 5 to 15 % by weight of a surfactant.

On the other hand, when the herbicide of the present invention is used in the form of a dust, generally, the dust can be prepared by mixing 1 to 15 % by weight of the pyrazole derivative and/or its salt of the present invention, 80 to 97 % by weight of a solid carrier and 2 to 5 % by weight of a surfactant. Further, when the herbicide of the present invention is used in the form of granules, the granules can be prepared by mixing 1 to 15 % by weight of the pyrazole derivative and/or its salt of the present invention, 90 to 97 % by weight of a solid carrier and 2 to 5 % by weight of a surfactant.

The above solid carrier is selected from mineral powders, and examples of the mineral powders include oxides such as diatomaceous earth and slaked lime, phosphates such as apatite, sulfates such as gypsum and silicates such as talc, pyrophyllite, clay, kaolin, bentonite, acidic terra alba, white carbon, powdered quartz and powdered silica.

The solvent is selected from organic solvents, and specific examples of the solvent include aromatic hydrocarbons such as benzene, toluene and xylene, chlorinated hydrocarbons such as o-chlorotoluene, trichloroethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

The surfactant is selected from anionic surfactants, nonionic surfactants, cationic surfactants and amphoteric surfactants (amino acid and betaine).

The herbicide of the present invention may contain, as an active ingredient, other herbicidally active component as required in addition to the pyrazole derivative of the general formula (I) and/or its salt. The "other" herbicidally active component includes conventionally known herbicides such as phenoxy-based, diphenyl ether-based, triazine-based, urea-based, carbamate-based, thiol carbamate-based, acid anilide-based, pyrazole-based, phosphoric acid-based, sulfonylurea-based and oxadiazone-based herbicides. The "other" herbicidally active component is properly selected from the above herbicides. The herbicide of the present invention may be used as a mixture with a pesticide, a fungicide, a plant growth regulator, a fertilizer, etc.

The novel pyrazole derivative of the formula (I), provided by the present invention, can be produced by the following method.

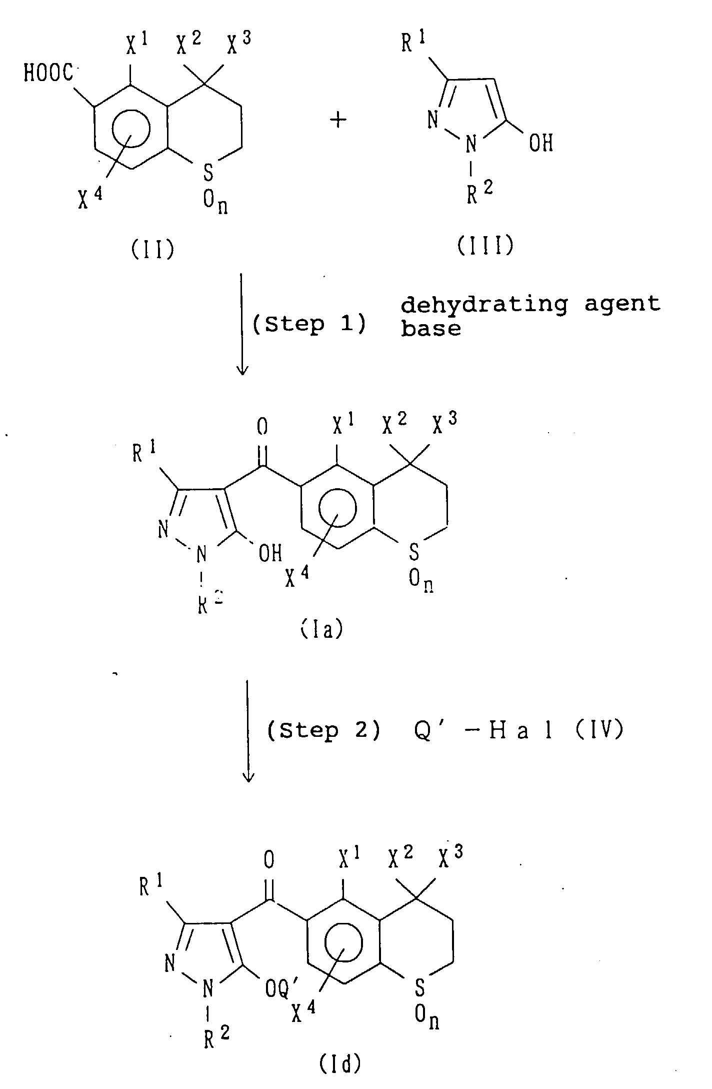

In the above reaction scheme, Q' is a group of -A-B, in which A is -SO₂-, -CO- or -CH₂CO-, and B is a C₁-C₈ alkyl group, a C₃-C₈ cycloalkyl group or a group of the formula (V), 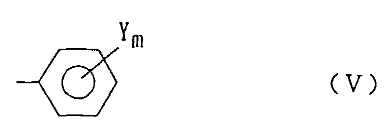 (in which Y is a halogen atom, a nitro group, a C₁-C₄ alkyl group, a C₁-C₄ alkoxy group or a halo C₁-C₄ alkyl group, and m shows the number of Y and is an integer of 0, 1 or 2), R¹, R², X¹, X², X³ and X⁴ are as already defined above, and Hal is a halogen atom.

Each step of the above production process will be explained below.

### (Step 1)

The compound of the formula (II) and the compound of the formula (III) are allowed to react in an inert solvent in the presence of a dehydrating agent, e.g., DCC (N,N'-dicyclohexylcarbodiimide, CDI (1,1-carbonyldiimidazole), EDC (1-(3-dimethylaminopropyl)-3-ethylcarbodiimide or the like and a base to produce the pyrazole derivative of the formula (Ia).

The amount of the compound of the formula (III) is preferably 1.0 to 3.0 mol per mole of the compound of the formula (II). The amount of the dehydrating agent is preferably 1.0 to 1.5 mol per mole of the compound of the formula (II). The base is not specially limited in kind, while potassium carbonate, sodium carbonate, or the like is preferably used in an amount of 0.5 to 2.0 mol per mole of the above compound of the formula (II). The inert solvent is not specially limited if it is inert to the reaction, while is preferably selected from acetonitrile, t-amyl alcohol, t-butyl alcohol and i-propyl alcohol. The reaction temperature can be set in the range of from room temperature to the boiling point of the solvent, while it is preferably around 80°C. The reaction time is 1 to 48 hours, while it is generally about 8 hours.

### (Step 2)

The compound (Ia) obtained in the step 1 is allowed to react with Q'-Hal (IV) (in which Q' and Hal are as already defined above) in the presence of a base to produce the compound (Id).

In the above step, the compound (Ia):compound (IV) molar ratio is preferably 1:1 to 1:3. Further, for collecting hydrogen halide produced as a byproduct by the reaction, it is preferred to use the base, such as sodium carbonate, potassium carbonate, triethylamine or pyridine, in an equimolar amount or more based on the starting material of the formula (Ia). The reaction temperature is preferably in the range of from room temperature to the boiling point of the solvent. The solvent used for the reaction is preferably selected from aromatic hydrocarbons such as benzene and toluene, ethers such as diethyl ether, ketones such as methyl ethyl ketone, and halogenated hydrocarbons such as methylene chloride and chloroform. Further, a two-phase solvent system of the above solvent and water may be used. In this case, a favorable result can be obtained by adding a phase transfer catalyst such as crown ether or benzyl triethylammonium chloride to the reaction system.

The aromatic carboxylic acid derivative of the formula (II) 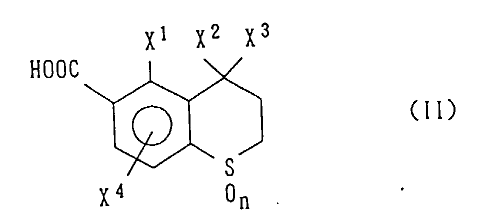 (wherein X¹ is a C₁-C₄ alkyl group or a halogen atom, each of X² and X³ is independently a C₁-C₄ alkyl group, X⁴ is a hydrogen atom, a halogen atom or a C₁-C₄ alkyl group, the position on which X⁴ is substituted is the 7- or 8-position, and n shows the number of oxygen and is an integer of 0, 1 or 2)
used for the reaction with the compound of the formula (III) is a novel compound which has not been disclosed in any literature, and is useful as an intermediate for the production of the pyrazole derivative of the present invention.

Specific examples of X¹, X², X³ and X⁴ in the above formula include those specified concerning the pyrazole derivative of the foregoing formula (I).

The aromatic carboxylic acid derivative of the formula (II) is an acidic substance, and can therefore be easily converted to a salt by treating it with a base. This salt is also included in the aromatic carboxylic acid derivative of the present invention. The above base is not specially limited if it is known. For example, it is selected from organic bases such as amines and anilines and inorganic bases such as sodium compounds and potassium compounds. The amines include a monoalkylamine, a dialkylamine and a trialkylamine. The alkyl group of the alkylamines is generally a C₁-C₄ alkyl group. The anilines include aniline, a monoalkylaniline and a dialkylaniline. The alkyl group of the alkylanilines is generally a C₁-C₄ alkyl group. The sodium compounds include sodium hydroxide and sodium carbonate. The potassium compounds include potassium hydroxide and potassium carbonate.

The aromatic carboxylic acid derivative of the formula (II) is produced by the following means (X), (Y) and (Z).

These means will be explained in detail hereinafter.
Means (X): Synthesis method in which X¹ = C₁-C₄ alkyl group, and X⁴ = C₁-C₄ alkyl group and is substituted on the 8-position.

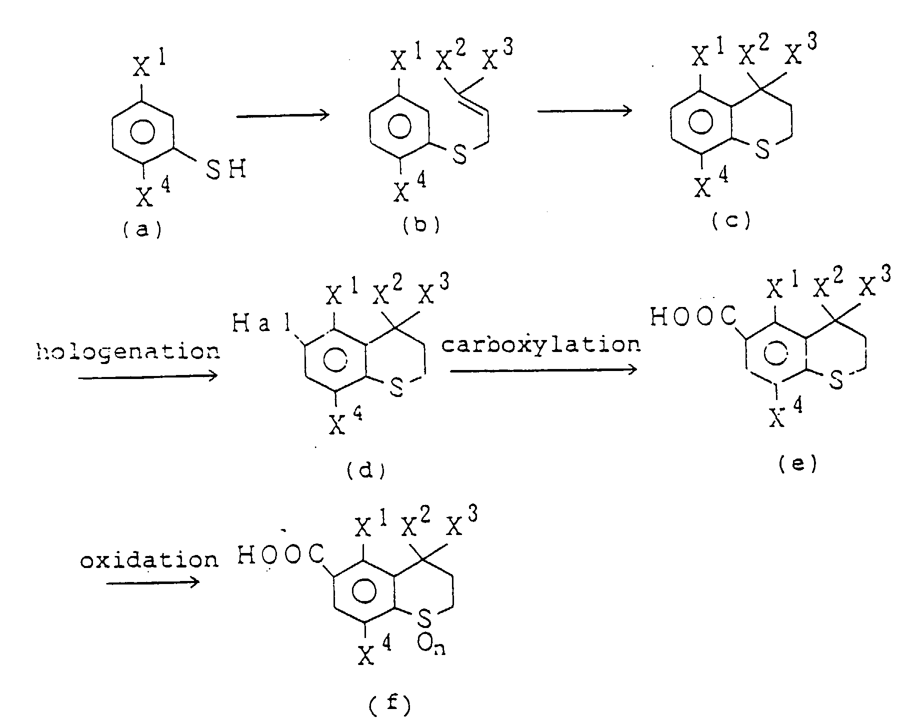

In the above reaction scheme, X², X³, n and Hal are the same as defined above.

2,5-Dialkyl thiophenol of the formula (a) which is a starting material is produced by a known method (e.g. New Handbook of Experimental Chemistry, Vol.14, Synthesis and Reaction of Organic Compounds, III, page 1704, Chapter 8.1, Thiols, f. It's Synthesis via Dithiocarbonate, published by Maruzen Publishing Co. Japan on February 20, 1986).

The starting material of the formula (a) and 3-halo-1,1-dialkylpropene (Hal-CH₂CH = CX²X³) are reacted in an inert solvent such as acetone, diethylether or dimethylformamide in the presence of a base such as anhydrous potassium carbonate, sodium hydroxide, potassium hydroxide, anhydrous sodium carbonate or triethylamine to produce the compound of the formula (b). 3-Halo-1,1-dialkylpropene and the base are used in amounts equivalent to 1.0 to 1.5 mol and 1.0 to 1.5 mol, respectively, per mole of the starting material of the formula (a). Preferably, the reaction temperature is generally 0 to 80°C, and the reaction time is generally approximately 1 to 8 hours.

The compound of the formula (b) is ring-closed by adding a dehydrative condensation agent such as polyphosphoric acid, sulfuric acid or phosphorus pentoxide, to obtain a compound of the formula (c) (thiochroman compound). The dehydrative condensation agent is used in an amount equivalent to 1 to 10 mol per mole of the compound of the formula (b). Preferably, the reaction temperature is generally 0 to 100°C, and the reaction time is generally approximately 1 to 8 hours.

The compound of the formula (c) is allowed to react with a halogenation reagent such as bromine, sulfuryl chloride or chlorine in the presence of a solvent such as methylene chloride, chloroform or carbon tetrachloride, to obtain a compound of the formula (d) in which halogen is substituted on the 6-position. Preferably, the reaction temperature is generally 0 to 80°C, and the reaction time is generally approximately 1 to 80 hours.

The compound of the formula (d) is allowed to react with magnesium (Mg) to form a Grignard reagent, and the Grignard reagent is allowed to react with carbon dioxide (CO₂) to obtain a compound (e) (n = 0, sulfide compound) which is an aromatic carboxylic acid derivative of the formula (II) provided by the present invention, in which a carboxyl group is substituted on the 6-position. As a solvent, it is preferred to use one selected from ethers such as diethyl ether and tetrahydrofuran. The reaction temperature is preferably 0 to 70°C, particularly preferably 20 to 60°C. The reaction time is generally approximately 1 to 7 hours.

The amount of the magnesium (Mg) for obtaining the Grignard reagent is preferably equivalent to 1.1 to 3.5 mol per mole of the compound of the formula (d). It is preferred to carry out the above Grignard reaction in the co-presence of alkyl iodide such as methyl iodide or alkyl bromide such as ethyl bromide, since the reaction smoothly proceeds. The amount of the alkyl halide used above is preferably equivalent to 0.1 to 2.5 mol per mole of the compound of the formula (d).

The reaction between the Grignard reagent and carbon dioxide (CO₂) is carried out by blowing a carbon dioxide gas from its cylinder or a carbon dioxide gas generated from dry ice (solid carbon dioxide) into the Grignard reagent in the solvent. Further, dry ice may be directly added to the Grignard reagent to allow these to react.

An oxidizing agent (e.g., hydrogen peroxide, peracetic acid or sodium permetaiodate) is allowed to react with the compound of the formula (e) in a solvent (e.g., acetic acid, water or methanol), to obtain a compound (f) (n = 1, sulfoxide compound / n = 2,-sulfone compound) which is an aromatic carboxylic acid derivative of the formula (II) provided by the present invention. When the amount of the oxidizing agent is 1 equivalent based on the compound (e), a sulfoxide (compound in which n = 1) is obtained, and when the amount of the oxidizing agent is 2 equivalent based on the compound (e), a sulfone (compound in which n = 2) is obtained.

Means (Y): Synthesis method in which X¹ = C₁-C₄ alkyl group and X⁴ = hydrogen atom or chlorine atom. 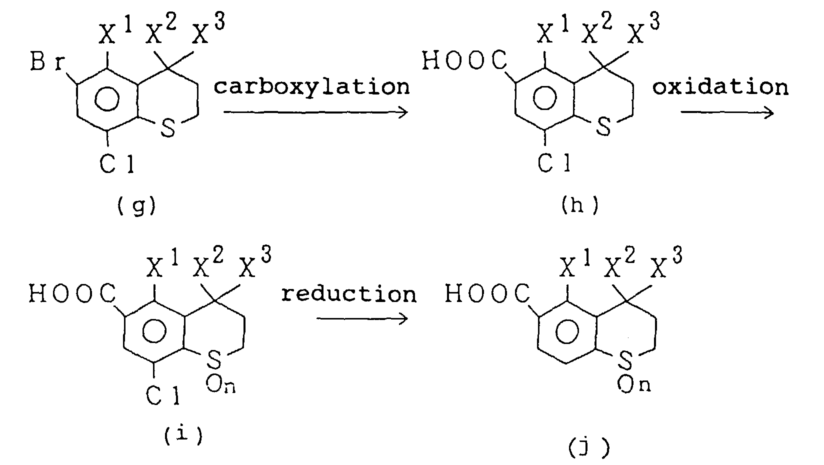 (wherein X², X³ and n are as already defined above.)

4,4,5-Trialkyl-6-bromo-8-chlorothiochroman of the formula (g) as a starting material can be obtained, for example, from the compound of the formula (a) in which X⁴ = chlorine in the above means (X) in the same manner as in means (X). That is, the 4,4,5-trialkyl-6-bromo-8-chlorothiochroman of the formula (g) corresponds to the compound of the formula (d) in which Hal is bromine and X⁴ is chlorine in means (X).

The compound of the formula (g) is allowed to react with magnesium (Mg) to form a Grignard reagent, and the Grignard reagent is allowed to react with carbon dioxide (CO₂) to obtain a compound (h) (n = 0, sulfide compound) which is an aromatic dicarboxylic acid derivative of the formula (II), provided by the present invention, in which a carboxyl group is introduced to the 6-position. The reaction conditions such as a solvent, the reaction temperature, the amount of magnesium used, are the same as those in the above means (X).

Then, an oxidizing agent is allowed to react with the compound of the formula (h) in a solvent to obtain a compound of the formula (i) (n = 1, sulfoxide compound / n = 2, sulfone compound) which is an aromatic carboxylic acid derivative of the formula (II). The reaction conditions such as the kind and amount of the oxidizing agent and the solvent are the same as those in the oxidation in the above means (X).

Finally, the compound of the formula (i) is reduced with a zinc powder, or the like in 60 % hydrous ethanol, to obtain a compound (j) which is an aromatic carboxylic acid derivative of the formula (II) provided by the present invention. Preferably, the reaction temperature is generally 20 to 120°C, and the reaction time is generally 1 to 12 hours.

### Means (Z): General method

The aromatic carboxylic acid derivative of the formula (II) can be also produced by the following steps in addition to means (X) and (Y).

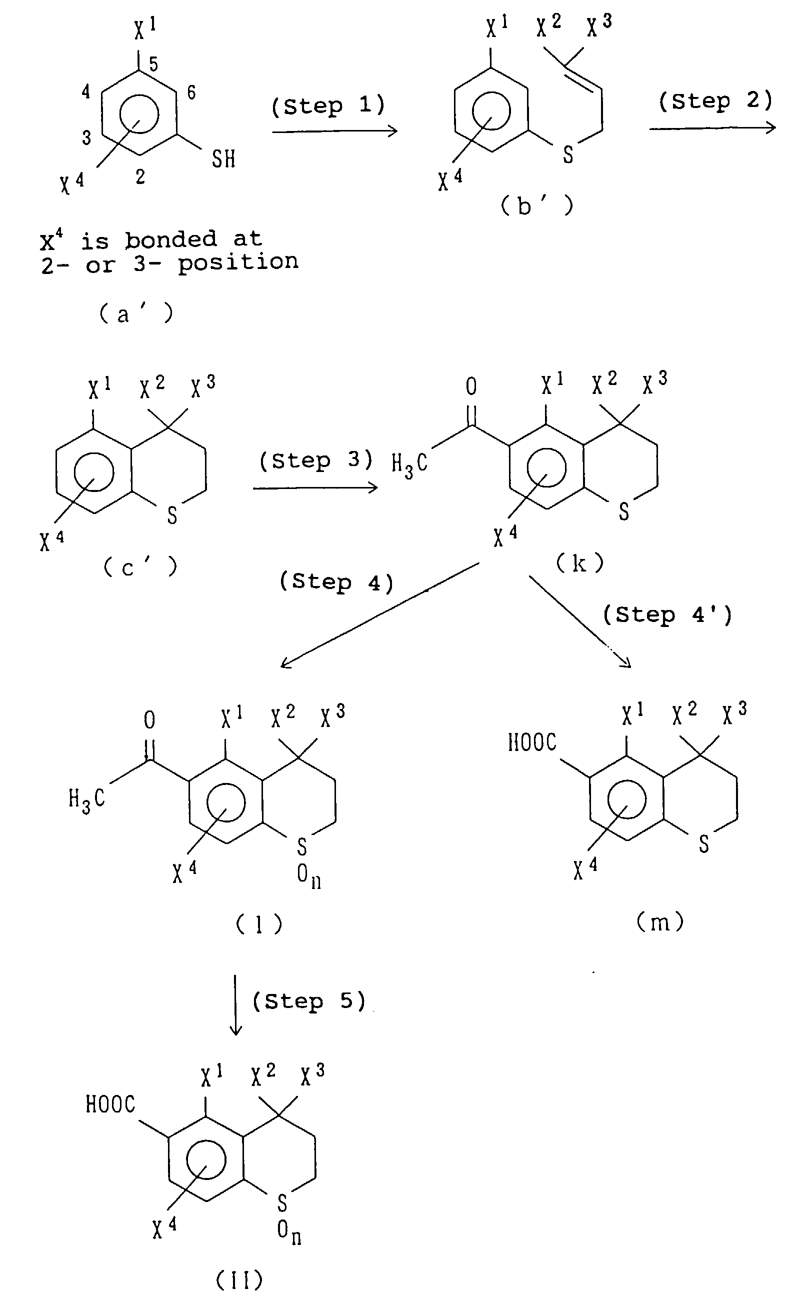

In the above reaction scheme, X¹, X², X³, X⁴ and n are as already defined above.

### (Steps 1 and 2)

The steps 1 and steps 2 for the production of a compound (c') from a starting material (a') through a compound (b') are the same as those for the production of the compound (c) from the compound (a) in means (X).

### (Step 3)

The compound of the formula (c') is allowed to react with acetyl chloride together with a Lewis acid such as aluminum chloride, zinc chloride or iron chloride or a proton acid such as hydrogen fluoride, sulfuric acid or phosphoric acid in the presence of a solvent such as dichloromethane, nitromethane, acetonitrile or benzene, to obtain a compound (k) in which an acetyl group is introduced to the 6-position. Each of the Lewis acid and the proton acid is used in an amount equivalent to 1.0 to 1.5 mol per mole of the compound (c'), and the acetyl chloride is used in an amount equivalent to 1.0 to 1.5 mol per mole of the compound (c'). Preferably, the reaction temperature is generally 0 to 80°C, and the reaction time is approximately 1 to 8 hours.

### (Step 4)

An oxidizing agent (e.g., hydrogen peroxide, peracetic acid or sodium permetaiodate) is allowed to react with the compound (k) (sulfide) in a solvent (e.g., acetic acid, water or methanol), to obtain a compound (1) (n = 1, sulfoxide compound / n = 2, sulfone compound). When the amount of the oxidizing agent is 1 equivalent based on the compound (k), a sulfoxide (compound in which n = 1) is obtained, and when the amount of the oxidizing agent is 2 equivalent based on the compound (k), a sulfone (compound in which n = 2) is obtained.

### (Step 4')

J. Am. Chem. Soc. 66, page 1,612 (1944) describes a method in which a methyl ketone group (acetyl group) on the 6-position of a thiochroman ring is converted to a carboxyl group without oxidizing a sulfur atom (S) of the thiochroman. That is, the methyl ketone compound of the formula (k) is allowed to react with iodine in pyridine and decomposed with an alkali, to give a compound (m) (n = 0, sulfide compound), which is an aromatic carboxylic acid derivative of the formula (II) provided by the present invention.

### (Step 5)

The methyl ketone compound (1) is converted to an aromatic carboxylic acid derivative (II) with an oxidizing agent (e.g., permanganate, chromic acid, halogen, oxygen or sulfuric acid).

### Examples

The present invention will be explained further in detail with reference to Examples hereinafter.

### Intermediate Preparation Example 1

4,4,5,8-Tetramethylthiochroman-6-carboxylic acid-1,1-dioxide used as a starting material in Preparation Example 1 to be described later was prepared in the following steps.

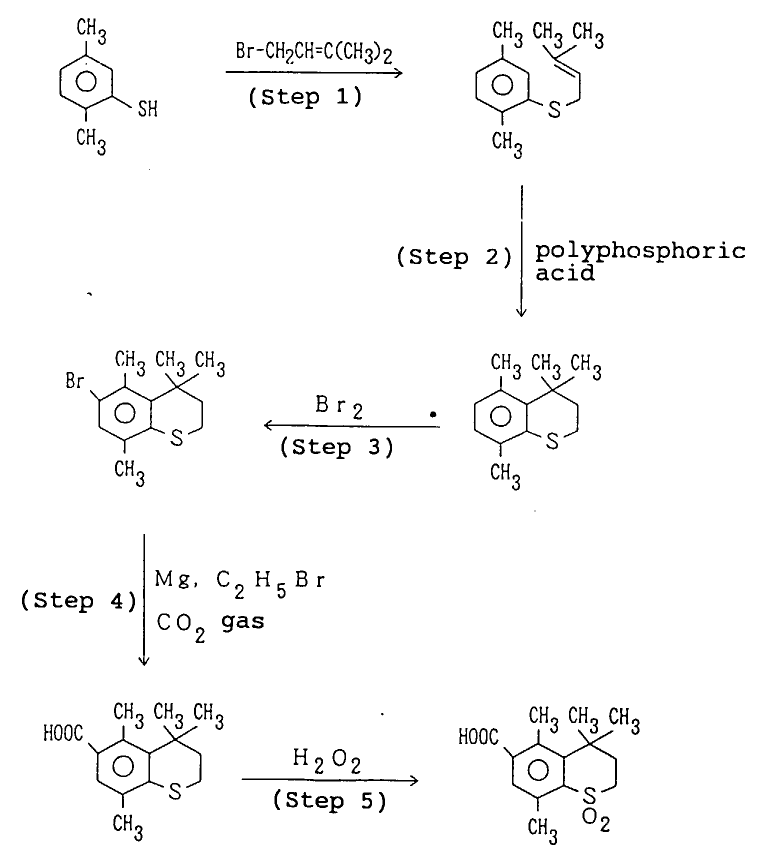

### Step (1)

Acetone in an amount of 80 ml, 9.1 g (0.06 mol) of 1-bromo-3-methyl-2-butene and 8.0 g (0.058 mol) of anhydrous potassium carbonate were added to 8.0 g (0.058 mol) of 2,5-dimethyl-thiophenol, and the mixture was heated under reflux for 1 hour. After the reaction, a formed salt was removed by filtration, and the acetone was distilled off under reduced pressure. Then, ethyl acetate was added to the resultant residue, and the mixture was washed with saturated sodium chloride aqueous solution and dried over anhydrous sodium sulfate. Then, ethyl acetate was distilled off under reduced pressure to give 12 g (yield 100 %) of 2-methyl-4-(2,5-dimethylphenyl)thio-2-butene.
N.M.R. (ppm, solvent: CDCl₃, internal standard: tetramethylsilane): 1.60 (3H,s), 1.70 (3H,s), 2.30 (6H,s), 3.50 (2H,d), 5.15 - 5.50 (H,m), 6.80 - 7.10 (3H,m)

### Step (2)

7.0 Grams (0.034 mol) of 2-methyl-4-(2,5-dimethylphenyl)thio-2-butene was added to 51 g of polyphosphoric acid with stirring. The mixture was further stirred at room temperature for 30 minutes, and the reaction mixture was poured into ice water, and extracted with n-hexane. An organic layer was washed with saturated sodium chloride aqueous solution and then dried over anhydrous sodium sulfate. The n-hexane was distilled off under reduced pressure to give 6.7 g (yield 95 %) of 4,4,5,8-tetramethyl-thiochroman.
N.M.R. (ppm, solvent: CDCl₃, internal standard: tetramethylsilane): 1.40 (6H,s), 1.95 - 2.15 (2H,m), 2.20 (3H,s), 2.50 (3H,s) 2.80 - 3.10 (2H,m), 6.80 (2H,dd)

### Step (3)

Methylene chloride in an amount of 100 ml was added to 13.2 g (0.064 mol) of 4,4,5,8-tetramethylthiochroman, 10.2 g (0.064 mol) of bromine was added dropwise at room temperature, and then, the mixture was allowed to react for 2 hours. After the reaction, 70 ml of a 2 % sodium hydrogensulfite aqueous solution was added, an excess of bromine was removed, and the mixture was liquid-separated. An organic layer was washed with a saturated sodium hydrogencarbonate aqueous solution and with a saturated sodium chloride aqueous solution, and dried over anhydrous sodium sulfate. Methylene chloride was distilled off under reduced pressure, and the resultant oily substance was purified by silica gel column chromatography to give 15.9 g (yield 87 %) of 4,4,5,8-tetramethyl-6-bromothiochroman.
N.M.R. (ppm, solvent: CDCl₃, internal standard: tetramethylsilane): 1.45 (6H,s), 1.90 - 2.10 (2H,m), 2.20 (3H,s), 2.50 (3H,s), 2.80 - 3.00 (2H,m), 7.20 (H,s)

### Step (4)

2.9 Gram (0.12 mol) of magnesium was dispersed in 100 ml of THF (tetrahydrofuran), and 7.4 g (0.068 mol) of ethyl bromide was added dropwise. After the mixture was allowed to react for 10 minutes, a solution of 9.7 g (0.034 mol) of 4,4,5,8-tetramethyl-6-bromothiochroman in THF was gradually added to the reaction mixture at room temperature. The reaction mixture was refluxed for 3 hours and then cooled to 10°C, and carbon dioxide gas was introduced by bubbling it for 1 hour. A 5 % hydrochloric acid in an amount of 100 ml was added to the reaction mixture to dissolve unreacted magnesium. THF was distilled off under reduced pressure, and methylene chloride was added to remove an aqueous layer. An organic layer was extracted with a 5 % potassium carbonate aqueous solution, and 5 % hydrochloric acid was added to the extract to adjust it to a pH of 1 to form a solid. The solid was recovered by filtration to give 7.4 g (yield 88 %) of 4,4,5, 8-tetramethylthiochroman-6-carboxylic acid.
N.M.R. (ppm, solvent: CDCl₃, internal standard: tetramethylsilane): 1.50 (6H,s), 1.90 - 2.10 (2H,m), 2.25 (3H,s), 2.70 (3H,s), 2.90 - 3.10 (2H,m), 7.55 (H,s)
I.R. (KBr tablet, cm⁻¹): 3,450 - 2,550, 2,980, 2,930, 1,695
m.p.: 166.0 - 167.1°C

### Step (5)

Acetic acid in an amount of 4 ml was added to 5 g (0.02 mol) of 4,4,5,8-tetramethylthiochroman-6-carboxylic acid. Further, 6.9 g (0.06 mol) of a 30 % hydrogen peroxide aqueous solution was added, and the mixture was heated at 80°C for 2 hours. A 2 % sodium hydrogensulfite aqueous solution in an amount of 50 ml was added to the reaction mixture to precipitate a solid, and the solid was recovered by filtration to give 5.1 g (yield 90 %) of 4,4,5,8-tetramethylthiochroman-6-carboxylic acid-1,1-dioxide.
N.M.R. (ppm, solvent: CDCl₃, internal standard: tetramethylsilane): 1.60 (6H,s), 2.25 - 2.50 (2H,m), 2.65 (3H,s), 2.75 (3H,s), 3.30 - 3.50 (2H,m), 7.55 (H,s)
I.R. (KBr tablet, cm⁻¹): 3,500 - 2,550, 3,000, 2,950, 1,630, 1,290, 1,130
m.p.: 208.8 ∼ 209.3°C

### Intermediate Preparation Example 2

4,4,5-Trimethylthiochroman-6-carboxylic acid-1,1-dioxide used as a starting material in Preparation Example 2 to be described later was prepared in the following steps.

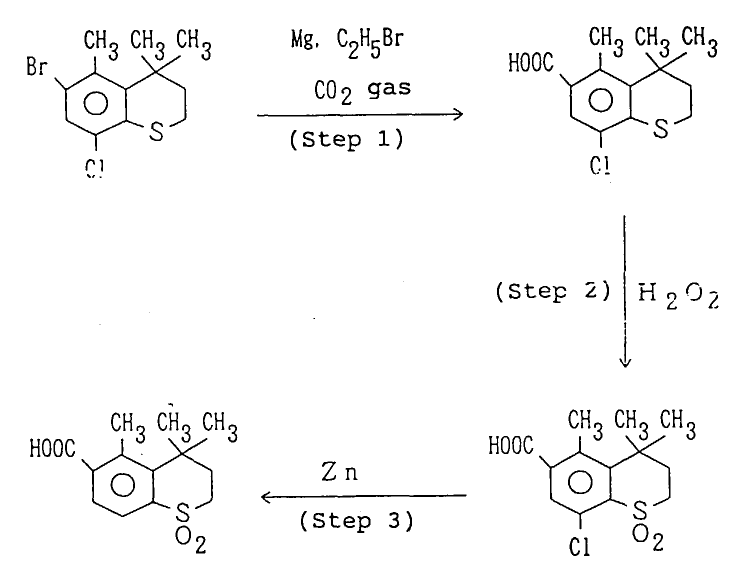

### Step (1)

0.63 Gram (0.026 mol) of magnesium was dispersed in 30 ml of THF, and 1.89 g (0.0173 mol) of ethyl bromide was added. After the mixture was allowed to react for 10 minutes, a solution of 1.76 g (0.0058 mol) of 4,4,5-trimethyl-6- bromo -8-thiochroman in THF was gradually added at room temperature. The reaction mixture was refluxed for 7 hours and then cooled to 10°C, and carbon dioxide gas was introduced by bubbling for 1 hour. A 5 % hydrochloric acid in an amount of 30 ml was added to the reaction mixture to dissolve unreacted magnesium. THF was distilled off under reduced pressure, and ethyl acetate was added to remove an aqueous layer. An organic layer was extracted with a saturated sodium bicarbonate aqueous solution, and 5 % hydrochloric acid was added to the extract to adjust it to a pH of 1 to form a solid. The solid was recovered by filtration to give 0.54 g (yield 40 %) of 4,4,5-trimethylthiochroman-6-carboxylic acid.
N.M.R. (ppm, solvent: CDCl₃, internal standard: tetramethylsilane): 1.50 (6H,s), 1.90 - 2.10 (2H,m), 2.60 (3H,s), 2.85 - 3.10 (2H,m), 7.55 (H,s)
I.R. (KBr tablet, cm⁻¹): 3,450 - 2,550, 2,980, 2,930, 1,695

### Step (2)

Acetic acid in an amount of 10 ml was added to 0.82 g (3.0 mmol) of 4,4,5-trimethyl-8-thiochroman-6-carboxylic acid. Further, 0.82 g (7.5 mmol) of a 30 % hydrogen peroxide aqueous solution was added, and the mixture was heated at 80°C for 3 hours. The reaction mixture was poured in 100 ml of ice water and extracted with ethyl acetate. An organic layer was washed with a saturated sodium chloride aqueous solution and dried over anhydrous sodium sulfate. The ethyl acetate was distilled off under reduced pressure to give 0.88 g (yield 96 %) of 4,4,5-trimethyl-8-chlorothiochroman-6-carboxylic acid-1 ,1 -dioxide.
N.M.R. (ppm, solvent: CDCl₃, internal standard: tetramethylsilane): 1.60 (6H,s), 2.25 - 2.50 (2H,m), 2.65 (3H,s), 3.30 - 3.50 (2H,m), 7.60 (H,s)
I.R. (KBr tablet, cm⁻¹): 3,500 - 2,550, 3,000, 2,950, 1,630, 1,290, 1,130

### Step (3)

0.88 Gram (2.90 mmol) of 4,4,5-trimethyl-8-thiochroman -6-carboxylic acid-1,1-dioxide was dissolved in 60 % hydrous ethanol, 0.55 q (8.70 mmol) of a zinc powder was added, and the mixture was heated under reflux for 5 hours. After the reaction, 50 ml of water was added, the zinc powder was removed by filtration, and the remaining reaction mixture was extracted with ethyl acetate. The extract was washed with 1 % hydrochloric acid and with a saturated sodium chloride aqueous solution, and dried over anhydrous sodium sulfate. The ethyl acetate was distilled off under reduced pressure to give 0.60 g (yield 78 %) of 4,4,5- trimethylthiochroman-6-carboxylic acid-1,1-dioxide.
N.M.R. (ppm, solvent: CDCl₃, internal standard: tetramethylsilane): 1.60 (6H,s), 2.30 - 2.50 (2H,m), 2.75 (3H,s), 3.30 - 3.50 (2H,m), 7.90 (2H,dd)

### Intermediate Preparation Example 3

5,8-Dichloro-4,4-dimethylthiochroman-6-carboxylic acid-1,1-dioxide used as a starting material in Preparation Example 3 to be described later was prepared in the following steps.

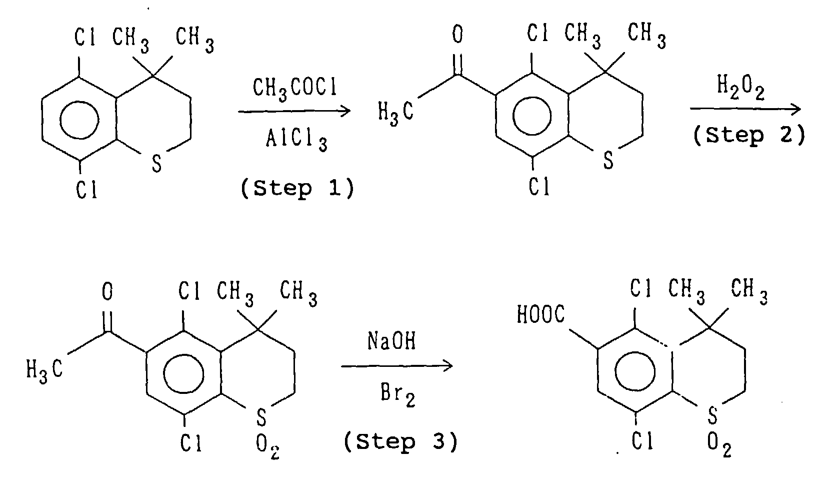

### Step (1)

1.26 Gram (9.45 mmol) of anhydrous aluminum chloride was suspended in 10 ml of methylene chloride, and 5 ml of a solution of 0.74 g (9.45 mmol) of acetyl chloride in methylene chloride was dropwise added with ice cooling. The mixture was stirred until a uniform reaction mixture was formed. Then, 5 ml of a solution of 1.95 g (7.88 mmol) of 5,8-dichloro-4,4-dimethylthiochroman in methylene chloride was added, and the mixture was allowed to react at room temperature for 1 hour. After the reaction, the reaction mixture was poured in ice water, and extracted with methylene chloride. An organic layer was washed with a saturated sodium chloride solution, and dried over anhydrous sodium sulfate. The methylene chloride was distilled off under reduced pressure, and the resultant oily substance was purified by silica gel column chromatography to give 1.27 g (yield 56 %) of 6-acetyl-5,8-dichloro-4,4- dimethylthiochroman.
N.M.R. (ppm, solvent: CDCl₃, internal standard: tetramethylsilane): 1.60 (6H,s), 1.95 - 2.20 (2H,m), 2.55 (3H,s), 2.80 - 3.10 (2H,m), 7.15 (H,s)

### Step (2)

Acetic acid in an amount of 1 ml was added to 1.22 g (4.22 mmol) of 6-acetyl-5,8-dichloro-4,4-dimethylthiochroman. Further, 1.43 g (12.66 mmol) of a 30 % hydrogen peroxide aqueous solution was added, and the mixture was heated at 80°C for 2 hours. A 2 % sodium hydrogensulfite solution in an amount of 10 ml was added to the reaction mixture to precipitate a solid, and the solid was recovered by filtration to give 0.96 g (yield 71 %) of 6-acetyl-5,8-dichloro-4,4-dimethylthiochroman-1,1-dioxide.
N.M.R. (ppm, solvent: CDCl₃, internal standard: tetramethylsilane): 1.65 (6H,s), 2.30 - 2.50 (2H,m), 2.55 (3H,s), 3.35 - 3.55 (2H,m), 7.30 (H,s)
I.R. (KBr tablet, cm⁻¹): 3,300 - 2,700, 1,700, 1.300, 1,130
m.p.: 154.3 - 155.1°C

### Step (3)

1.04 Gram (26.1 mmol) of sodium hydroxide was dissolved in 20 ml of water, 0.5 ml (8.7 mmol) of bromine was added with ice cooling. Further, 0.93 g (2.9 mmol) of 6-acetyl-5,8-dichloro-4,4-dimethylthiochroman-1,1-dioxide was added, and the mixture was stirred for 3 hours, and then heated at 100°C for 5 hours. After the reaction, ethyl acetate was added, and the mixture was liquid-separated. Hydrochloric acid was added to the aqueous layer to adjust it to a pH of 1. The aqueous layer was extracted by adding ethyl acetate, and an organic layer was dried over anhydrous sodium sulfate. The ethyl acetate was distilled off under reduced pressure to give 0.84 g (yield 90 %) of 5,8-dichloro-4,4-dimethylthiochroman-6-carboxylic acid-1,1-dioxide.
N.M.R. (ppm, solvent: deutero, internal standard: tetramethylsilane): 1.70 (6H,s), 2.30 - 2.50 (2H,m), 3.40 - 3.60 (2H,m), 7.75 (H,s)
I.R. (KBr tablet, cm⁻¹): 3,500 - 2,500, 1,750, 1,300, 1,130

### Intermediate Preparation Example 4

5-Chloro-4,4,8-trimethyl-thiochroman-6-carboxylic acid-1,1-dioxide used as a starting material in Preparation Example 29 to be described later was prepared in the following steps.

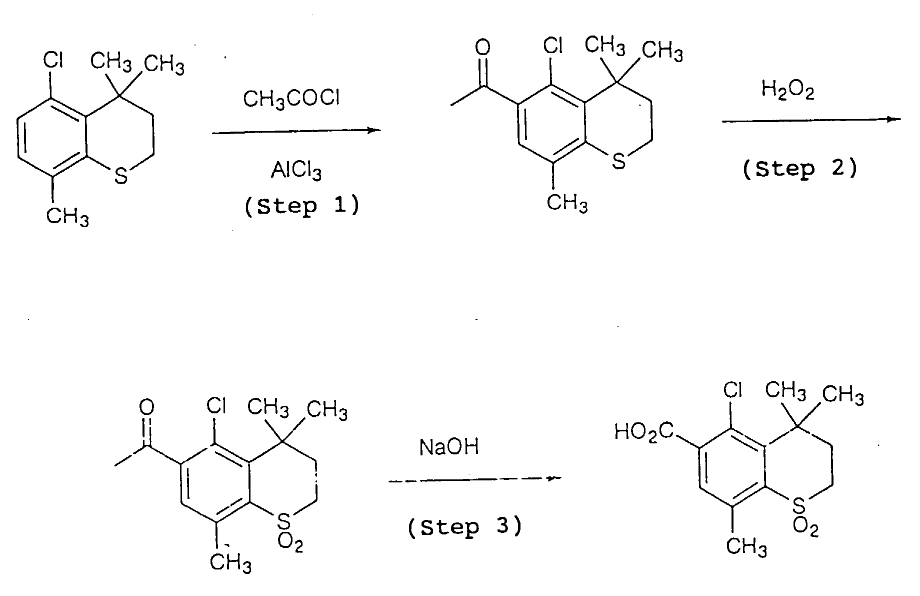

### Step (1)

3.2 Grams (24 mmol) of anhydrous aluminum chloride was suspended in 20 ml of methylene chloride, and 10 ml of a solution of 1.9 g (24 mmol) of acetyl chloride in methylene chloride was added with ice cooling. The mixture was stirred until a uniform reaction mixture was formed. Then, 10 ml of a solution of 4.5 g (20 mmol) of 5-chloro-4,4,8-trimethyl thiochroman in methylene chloride was added, and the mixture was allowed to react at room temperature for 1 hour. After the reaction, the reaction mixture was poured in ice water, and extracted with methylene chloride. The organic layer was washed with a saturated sodium chloride aqueous solution and dried over anhydrous sodium sulfate. The methylene chloride was distilled off under reduced pressure, and the resultant oily substance was purified by silica gel chromatography to give 3.0 g (yield 56 %) of 6-acetyl-4-chloro-4,4, 8-trimethylthiochroman.

### Step (2)

Acetic acid in an amount of 5 ml was added to 3.0 g (11 mmol) of 6-acetyl-5-chloro-4,4,8-trimethylthiochroman. Further, 3.7 g (33 mmol) of a 30 % hydrogen peroxide aqueous solution was added, and the mixture was heated at 80°C for 2 hours. A 2 % sodium hydrogensulfite aqueous solution in an amount of 20 ml was added to the reaction mixture to precipitate a solid, and the solid was recovered by filtration to give 2.8 g (yield 85 %) of 6-acetyl-5-chloro-4,4,8-trimethylthiochroman-1,1-dioxide.
N.M.R. (ppm, solvent: CDCl₃, internal standard: tetramethylsilane): 1.67 (6H,s), 2.30 - 2.50 (2H,m), 2.56 (3H,s), 2.76 (3H,s), 3.30 - 3.50 (2H,m), 7.05 (H,s)

### Step (3)

3.6 Grams (85 mmol) of sodium hydroxide was dissolved in 70 ml of water, and 1.5 ml (29 mmol) of bromine was added with ice cooling. Further, 2.8 g (9.3 mmol) of 6-acetyl-5-chloro-4,4,8-trimethylthiochroman-1,1-dioxide was added, and the mixture was stirred for 3 hours and then heated at 100°C for 5 hours. After the reaction, ethyl acetate was added, and the mixture was liquid-separated. Hydrochloric acid was added to the aqueous layer to adjust it to a pH of 1. The aqueous layer was extracted by adding ethyl acetate, and the organic layer was dried over anhydrous sodium sulfate. The ethyl acetate was distilled off under reduced pressure to give 2.1 g (yield 75 %) of 5-chloro-4,4,8 trimethylthiochroman-6-carboxylic acid-1,1-dioxide.
N.M.R. (ppm, solvent: deutero acetone, internal standard: tetramethylsilane): 1.70 (6H,s), 2.30 - 2.50 (2H,m), 2.75 (3H,s), 3.40 - 3.60 (2H,m), 7.50 (H,s)

Preparation Examples of the novel pyrazole derivative to achieve the first object of the present invention will be described hereinafter.

### Preparation Example 1

7.4 Grams (0.026 mol) of the 4,4,5,8-tetramethyl thiochroman-6-carboxylic acid-1,1-dioxide obtained in Intermediate Preparation Example 1, 3.4 g (0.03 mol) of 1-ethyl-5-hydroxypyrazole and 6.22 g (0.03 mol) of DCC (N,N'-dicyclohexylcarbodiimide) were added to 50 ml of tert-amyl alcohol at once, and the mixture was stirred at room temperature for 30 minutes. Then, 1.8 g (0.013 mol) of anhydrous potassium carbonate was added. The reaction mixture was allowed to react at 80°C for 8 hours, and then, the reaction solvent was distilled off under reduced pressure. The resultant residue was dispersed in a 5 % potassium carbonate aqueous solution and ethyl acetate, to separate it into two layers. Further, the aqueous layer was adjusted with 5 % hydrochloric acid to a pH of 1 to form a solid, and the solid was recovered by filtration to give 6.13 g (yield 62 %) of 4,4,5,8-tetramethyl-6-(1-ethyl-5-hydroxypyrazol-4-yl) carbonylthiochroman-1,1-dioxide.

### Preparation Examples 2 - 5

Compounds shown in the right column of Table 1 were obtained in the same manner as in Preparation Example 1 except that the 4,4,5,8-tetramethylthiochroman-6-carboxylic acid -1, 1-dioxide was replaced with raw materials shown in the left column of Table 1.

### Preparation Example 6

4,4,5,8-Tetramethyl-6-(1,3-dimethyl-5-hydroxypyrazol-4-yl)carb onylthiochroman-1,1-dioxide in an amount of 0.46 g (yield 70 %) was obtained in the same manner as in Preparation Example 1 except that the 1-ethyl-5-hydroxypyrazole was replaced with 1,3-dimethyl-5-hydroxypyrazole.

Table 1 shows the raw materials used in Preparation Examples 1 to 6, structural formulae and yields of the compounds obtained in Preparation Examples 1 to 6. Table 2 shows their physical properties.

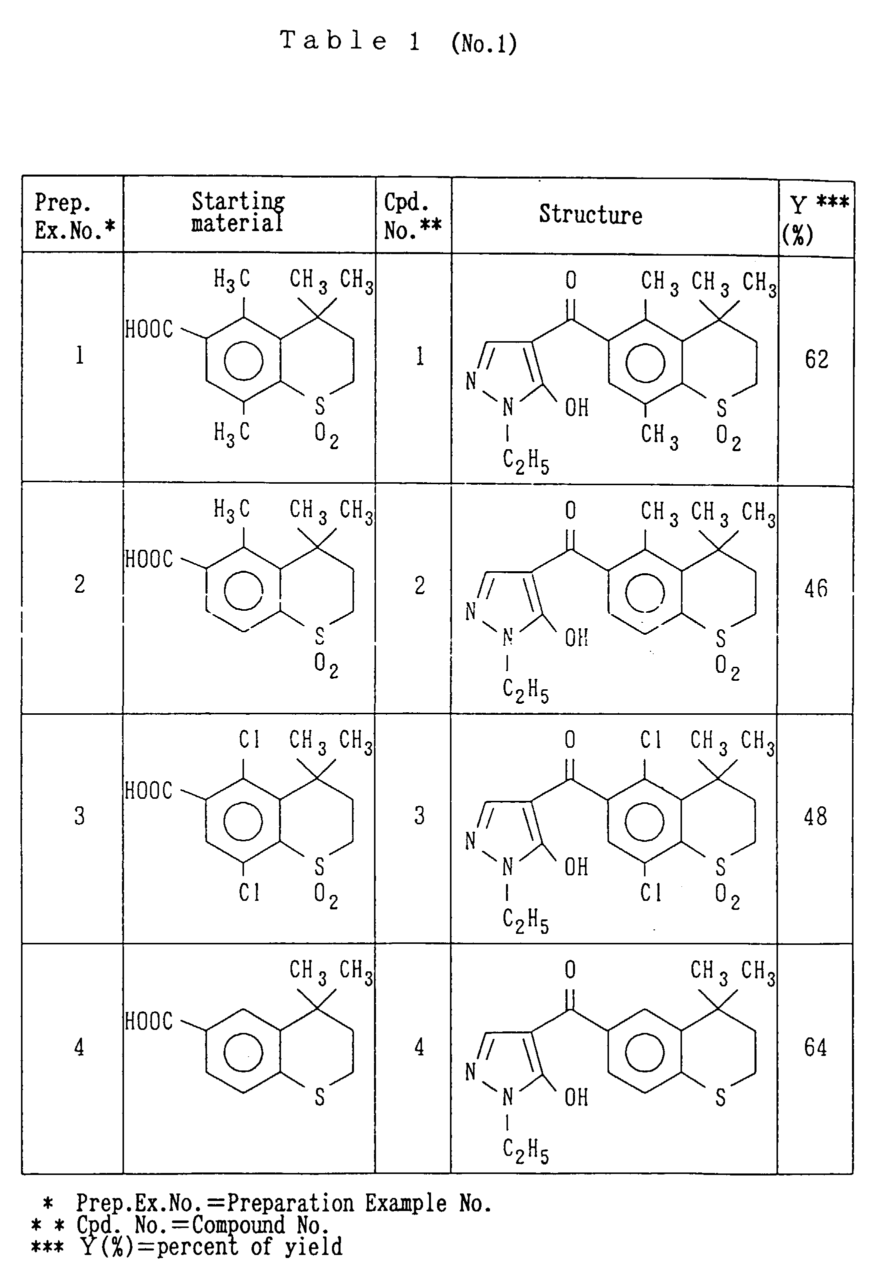

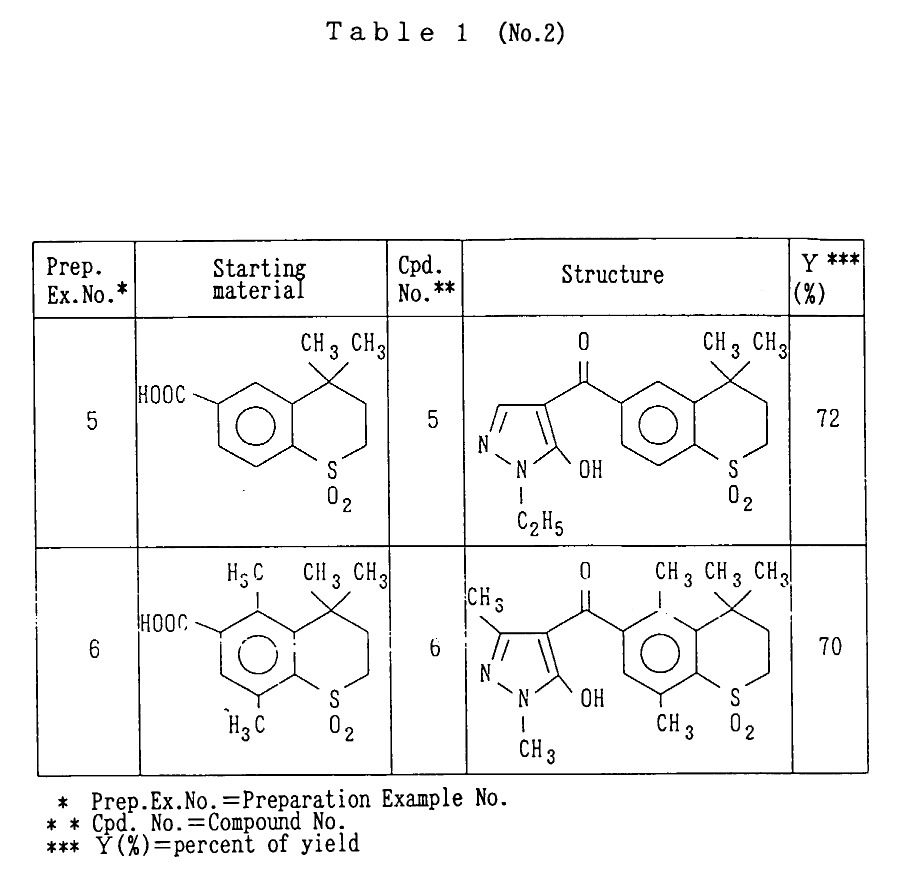

**Table 2**

| Prep. Ex.No.* | Cpd. No.** | NMR (ppm) Int.Std.*** tetramethylsilane Solvent deuterochroroform | I.R. (cm⁻¹) KBr tablet | mp (°C) |
|---|---|---|---|---|
| 1 | 1 | 1.45(3H, t)1.55(6H, s)2.30-2.50(2H, m) 2.50(3H, s)2.80(3H, s)3.40-3.60(2H, m) 4.10(2H, q)6.20(H, s)7.20(H, s) | 2550-3500, 2950, 3000, 1630, 1290, 1130 | 208.8-209.3 |
| 2 | 2 | 1.38(3H, t)1.60(6H, s)2.38-2.55(2H, m) 2.70(3H, s)3.40 3.54(2H, m)4.00(2H, q) 7.30(H, s)7.45(H, d)7.80(H, d) ( deuteroacetone as solvent ) | 2550-3500, 2950, 3000, 1620, 1290, 1130 | glass-like |
| 3 | 3 | 1.48(3H, t)1.70(6H, s)2.20-2.42(2H, m) 3.40-3.60(2H, m)4.08(2H, q)7.25(H, s) 7.42(H, s) | 2500-3500, 2950, 3000, 1660, 1320, 1160 | 263.7-263.9 |
| 4 | 4 | 1.38(6H, s)1.46(3H, t)1.90-2.10(2H, m) 3.00-3.20(2H, m)4.10(2H, q)7.20(H, d) 7.60(H, dd)7.76(H, s)7.95(H, d) | not measured | glass-like |
| 5 | 5 | 1.40(3H,t)1.46(6H, s)2.35-2.50(2H, m) 3.45.-3.60(2H, m)4.05(2H, q)7.68(H, s) 7.86-8.06(3H, m) ( deuteromethanol as solvent ) | not measured | glass-like |
| 6 | 6 | 1.55(6H, s)1.65(3H, s)2.30-2.50(2H, m) 2.45(3H, s)2.80(3H, s)3.30-3.50(2H, m) 3.65(3H, s)7.00(H, s) | 2570-3700, 2950, 1630, 1290, 1130 | glass-like |

| | | | | |
|---|---|---|---|---|
| * Prep.Ex.No. =Preparation Example No. | | | | |
| ** Cpd. No. =Compound No. | | | | |
| *** Int.Std. =Internal Standard | | | | |

### Preparation Example 7

0.70 Gram (1.9 mmol) of the 4,4,5,8-tetramethyl-6-(1-ethyl-5-hydroxypyrazol-4-yl)carbonylthiochroman-1,1-dioxide obtained in Preparation Example 1 was dissolved in 8 ml of methylene chloride. Then, a solution of 0.51 g (3.8 mmol) of potassium carbonate in 5 ml of water was added, and further, 0.43 g (3.8 mmol) of methanesulfonyl chloride and 0.05 g (0.2 mmol) of benzyl triethyl- ammonium chloride were added. The mixture was allowed to react at room temperature for 2 hours, and then heated under reflux for 2 hours. After the reaction mixture was allowed to cool, a methylene chloride layer was separated and recovered, and it was dried over anhydrous sodium sulfate. The solvent was distilled off under reduced pressure, and the resultant oil was purified by silica gel chromatography, to give 0.41 g (yield 49 %) of 4,4,5,8-tetramethyl-6-(1-ethyl-5-methanesulfonyloxypyrazol-4-yl)carbon ylthiochroman-1,1-dioxide (Compound 7).

### Preparation Examples 8 - 17

Compounds shown in the right column of Table 3 were obtained in the same manner as in Preparation Example 7 except that the methanesulfonyl chloride was replaced with reaction reagents shown in the left column of Table 3.

### Preparation Example 18

0.5 Gram (1.3 mmol) of the 4,4,5,8-tetramethyl-6-(1-ethyl-5-hydroxypyrazol-4-yl)carbonylthiochroman-1,1-dioxide obtained in Preparation Example 1 was dissolved in 10 ml of methylene chloride, and 0.27 g (2.6 mmol) of triethylamine and 0.21 g (2.6 mmol) of acetyl chloride were added. The mixture was allowed to react at room temperature for 8 hours. Water was added to the reaction mixture, and the methylene chloride layer was separated and dried over anhydrous sodium sulfate. The solvent was distilled off under reduced pressure, and the resultant oil was purified by silica gel chromatography, to give 0.32 g (yield 58 %) of 4,4,5,8-tetra-methyl-6-(1-ethyl-5-acetoxypyrazol-4-yl)carbonylthiochroman-1, 1-dioxide (Compound 18).

### Preparation Examples 19 - 23

Compounds 19 to 23 shown in the right column of Table 3 were obtained in the same manner as in Preparation Example 18 except that the acetyl chloride was replaced with reaction reagents shown in the left column of Table 3.

### Preparation Example 24

0.5 Gram (1.3 mmol) of the 4,4,5,8-tetramethyl-6-(1-ethyl-5-hydroxypyrazol-4-yl)carbonylthiochroman-1,1-dioxide obtained in Preparation Example 1 was dissolved in 10 ml of methyl ethyl ketone, and 0.14 g (1.4 mmol) of chloroacetone and 0.37 g (2.6 mmol) of potassium carbonate were added. The mixture was heated under reflux for 4 hours. The solvent was distilled off under reduced pressure, and ethyl acetate was added to the resultant residue. The residue was washed with a saturated sodium bicarbonate aqueous solution. The organic layer was dried over anhydrous sodium sulfate, and then the solvent was distilled off under reduced pressure. The resultant oil was purified by silica gel chromatography, to give 0.42 g (yield 73 %) of Compound 24.

### Preparation Example 25

Compound 25 in an amount of 0.61 g (yield 92 %) was obtained in the same manner as in Preparation Example 24 except that the chloroacetone was replaced with phenacyl bromide.

Table 3 shows the structures and yields of Compounds 7 to 25 obtained in Preparation Examples 7 to 25. Table 4 shows their physical properties.

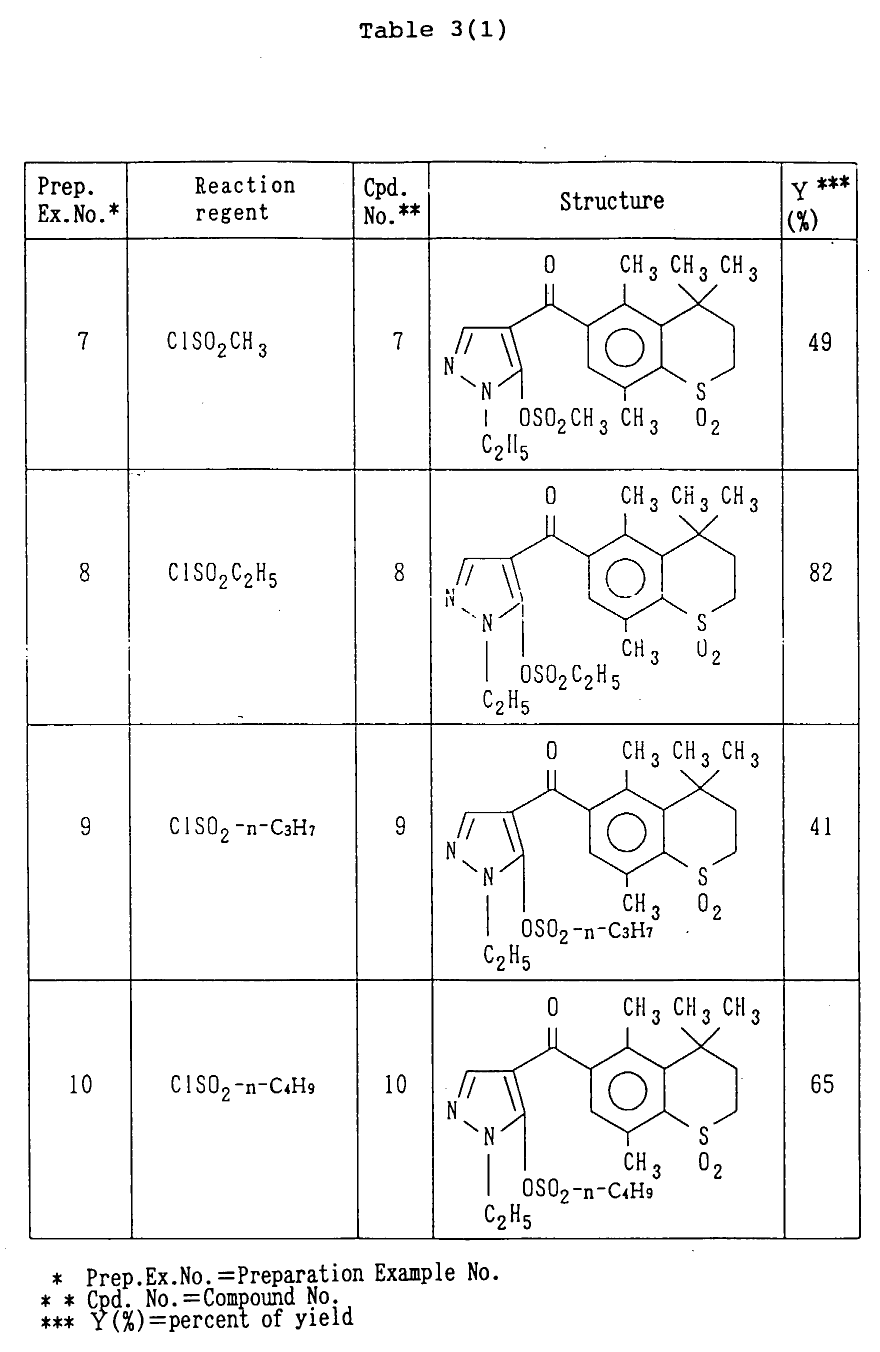

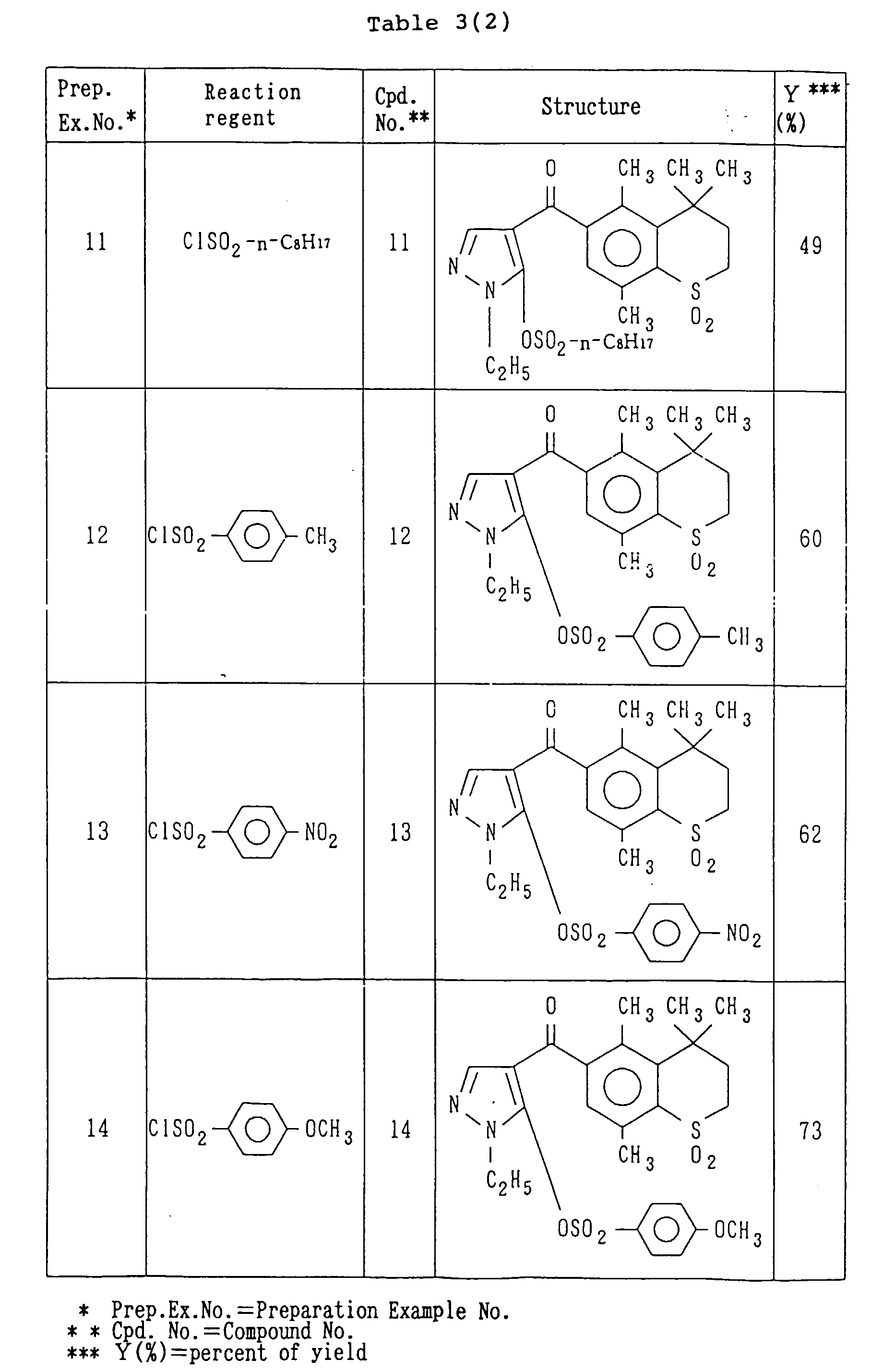

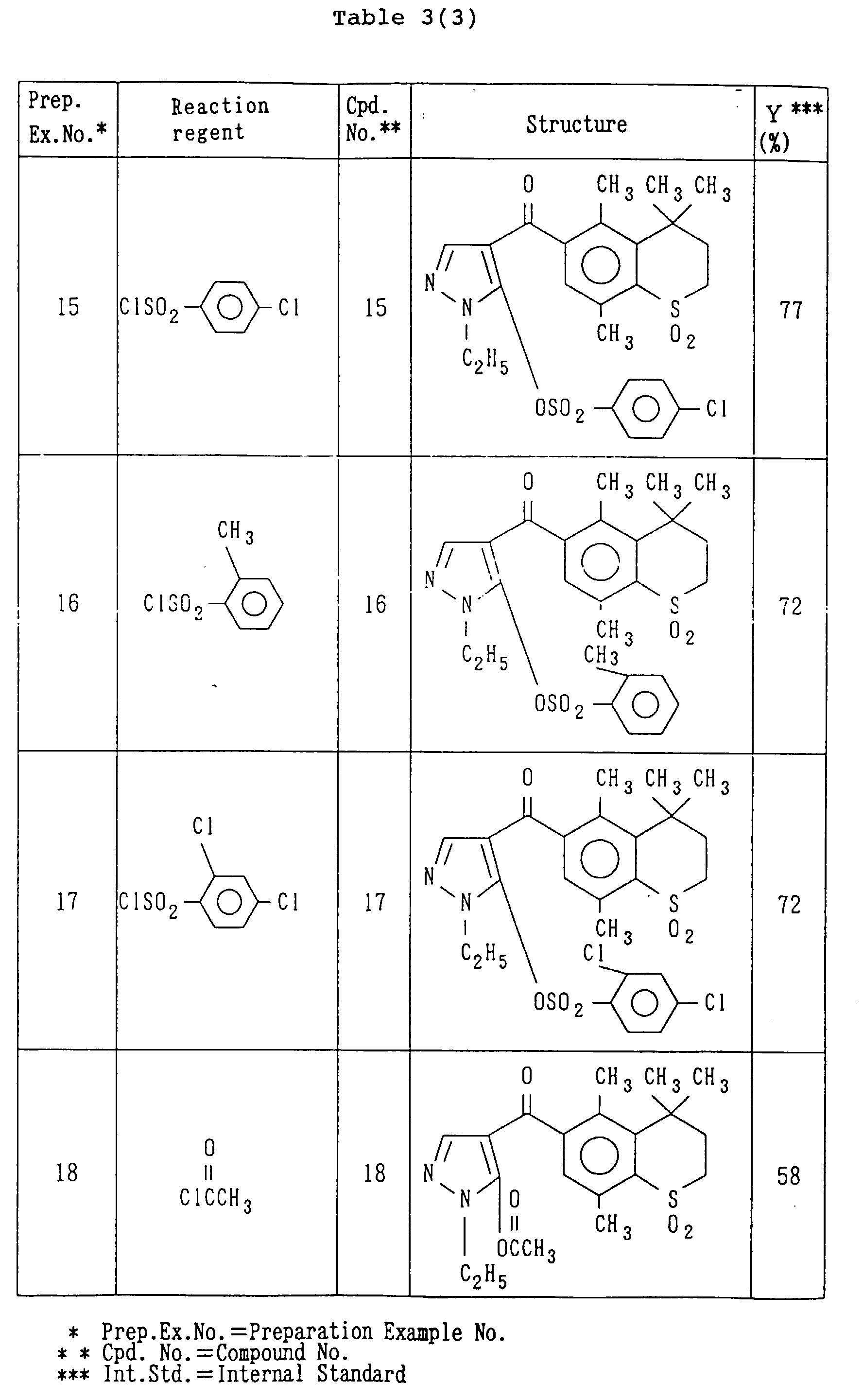

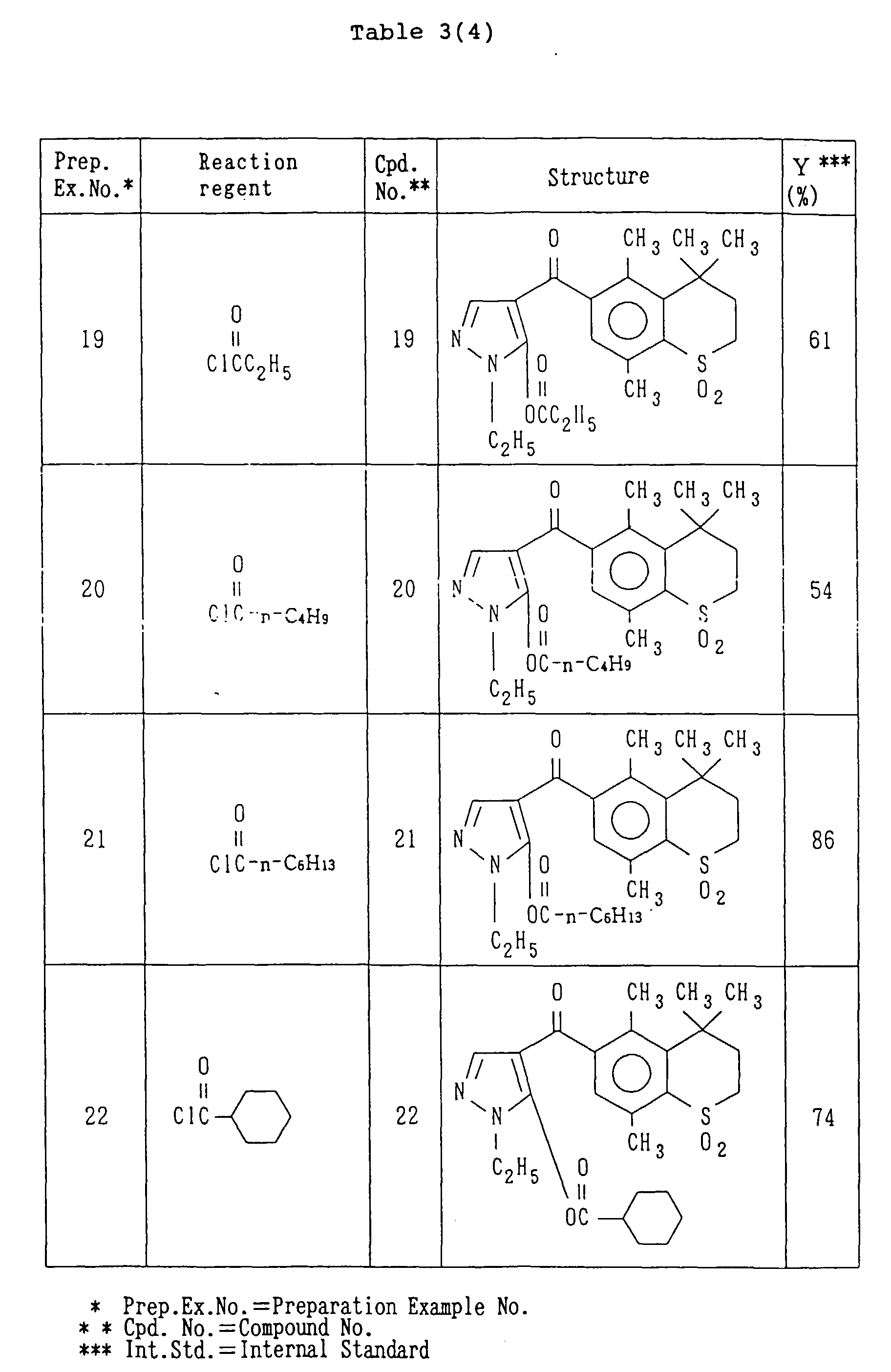

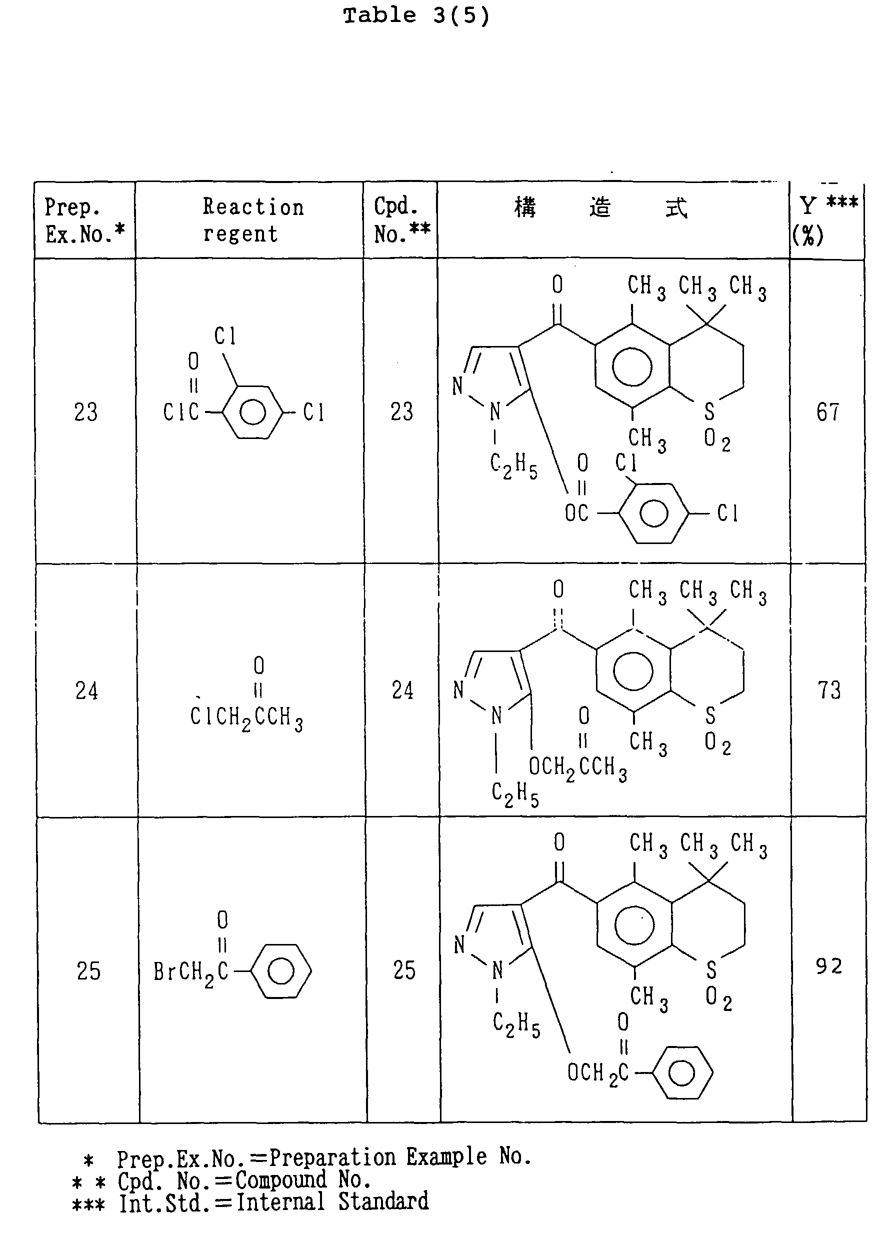

**Table 4 (No.1)**

| Prep. EX.No.* | Cpd. No.** | NMR (ppm) Int.Std.*** tetramethylsilane Solvent deuterochroroform | I.R.(cm⁻¹) KBr tablet | mp (°C) |
|---|---|---|---|---|
| 7 | 7 | 1.55(3H, t)1.60(6H, s)2.30-2.60(2H, m) 2.50(3H, s)2.80(3H, s)3.30-3.60(2H, m) 3.65(3H s)4.25(2H, q)7.10(H, s) 7.40(H, s) | 2980, 1665. 1140, 1300, 1200, 1390 | 202.2-203.4 |
| 8 | 8 | 1.50(3H, t)1.60(6H, s)1.70(3H, t) 2.30-2.60(2H, m) 2.50(3H, s)2.80(3H, s) 3.30-3.60(2H, m)3.80(2H, q)4.20(2H, q) 7.10(H, s)7.40(H, s) | 2940, 3000, 1660, 1180, 1140, 1290, 1380 | 164.1-165.7 |
| 9 | 9 | 1.20(3H, t)1.55(3H, t)1.60(6H, s) 2.00-2.50(4H, m)2.50(3H, s)2.80(3H, s) 3.30-3.60(2H, m)8.60-3.90(2H, m) 4.20(2H, q)7.10(H, s)7.40(H, s) | 2970, 3000, 1680, 1140, 1300, 1190, 1390 | glass-like |
| 10 | 10 | 1.05(3H, t)1.40-1.80(5H, m)1.55(6H, s) 1.90-2.20(2H, m)2.25-2.50(2H, m)2.50 (3H, s)2.80(3H, s) 3.30-3.60(2H, m) 3.65 3.90(2H, m)4.20(2H, q)7.10(H, s) 7.40(H, s) | 2900, 2980, 1660, 1130, 1300, 1180. 1380 | 177.9-179.1 |
| 11 | 11 | 0.80-1.00(3H, m) 1.20-1.80(13H, m) 1.60(6H, s)1.90-2.20(2H, m)2.25-2.50 (2H, m)2.50(3H, s) 2.80(3H, s)3.30-3.60 (2H, m) 3.65-3.90 (2H, m)4.20(2H, q) 7. 10(H, s)7. 40(H, s) | 2880, 2950, 1670, 1140, 1300, 1380, 1390 | glass-like |
| 12 | 12 | 1.50(3H, t)1.60(6H, s)2.20-2.60(2H, m) 2.40(3H, s)2.50(3H, s)2.77(3H, s) 3.30-3.60(2H, m)4.20(2H, q) 6.90(H, s) 7.40(2H, d)7.48(H, s)7.90(2H, d) | 2950, 3000, 1670, 1130, 1300, 1180, 1380 | 164.9-166.3 |
| 13 | 13 | 1.57(3H, t)1.60(6H, s)2.20-2.50(2H, m) 2.40(3H, s)2.80(3H, s)3.30-3.57(2H, m) 4.25(2H, q) 6.95(H, s)7.40(H, s) 8.40(4H, dd) | 2950, 1665, 1130, 1290, 1200, 1380 | 172.3-174.2 |
| 14 | 14 | 1.50(3H, t)1.60(6H, s)2.20-2.50(2H, m) 2.40(3H, s)2.78(3H, s)3.30-3.50(2H, m) 3.90(3H, s)4.20(2H, q)6.90(H, s)7.10 (2H, d)7.48(H, s)7.90(2H, d) | 2950, 3000, 1660, 1100, 1290, 1180, 1380 | 174.9-176.7 |
| 15 | 15 | 1.50(3H, t)1.57(6H, s)2.25-2.60(2H, m) 2.40(3H, s)2. 78(3H, s)3.30-3.60(2H, m) 4.20(2H, q) 6.90(H, s)7.45(H, s)7.60 (2H, d) 8.00(2H, d) | 2980, 3000, 1680, 1140, 1300, 1200. 1400 | 145.3-147.8 |
| 16 | 16 | 1.50(3H, t)1.53(6H, s)2.20-2.60(2H, m) 2.30(3H, s)2.73(3H, s)2. 88(3H, s) 3.30-3.60(2H, m)4.20(2H, q) 6.80(H, s) 7. 25(H, s)7.30-8.00(4H, m) | 2950, 3000, 1680, 1130, 1290, 1200, 1390 | 182.1-184.5 |

| | | | | |
|---|---|---|---|---|
| * Prep. Ex. No. = Preparation Example No. | | | | |
| **Cpd. No. = Compound No. | | | | |
| *** Int.Std. = Internal Standard | | | | |

**Table 4 (No.2)**

| Prep. Ex.No.* | Cpd. No.** | NMR (ppm) Int.Std.*** tetramethylsilane Solvent deuterochroroform | I.R.(cm⁻¹) KBr tablet | mp (°C) |
|---|---|---|---|---|
| 17 | 17 | 1.55(3H, t)1.60(6H,s)2.20-2.60(2H, m) 2.30(3H, s)2.75(3H, m)3.28-3.50 (2H, m) 4.25(2H, q)6. 90(H, s)7.25(H, s) 7.48-7. 78(2H, m)7.85-8.00(H, m) | 2950, 3000, 1670, 1130, 1290, 1190, 1400 | 181.8-185.8 |
| 18 | 18 | 1.45(3H, t)1.55(6H, s)2.20-2.60(2H, m) 2.25(3H, s)2.45(3H, s)2.79(3H, s) 3.27-3.58(2H, m)4.00(2H, q)7.00(H, s) 7.60(H, s) | 2950, 2980, 1660, 1120, 1160, 1290 | 187.7-191.4 |
| 19 | 19 | 1.22(3H, t)1.47(3H, t)1.60(6H, s) 2.20-2.70(4H, m)2.45(3H, s)2.80(3H, s) 3.30-3.55(2H, m)4.00(2H, q)7.05(H, s) 7.60(H, s) | 2950, 3000, 1660, 1110, 1300, 1200, 1410 | 178.0-179.6 |
| 20 | 20 | 0.98(3H, t)1.20-2 00(7H, m)1.58(6H, s) 2.20 2.70(4H, m)2.45(3H, s)2.73(3H, s) 3.30-3.55(2H, m)4.00(2H, q)7.05(H, s) 7.57(H, s) | 2880, 2980, 1670, 1140, 1300 | glass-like |
| 21 | 21 | 0.90(3H, t)1.10-1.90(11H, m)1.55(6H, s) 2.20-2.70(4H, m)2.48(3H, m)2.75(3H, s) 3.30-3.57(2H, m) 4.00(2H, q)7.03(H, s) 7.55(H, s) | 2880, 2950, 1660, 1120, 1800.1190 | glass-like |
| 22 | 22 | 1.42(3H, t)1.20-2.20(11H, m)1.55(6H, s) 2.25-2.65(2H, m)2.42(3H, s)2.80(3H, s) 3.30-3.55(2H, m)3. 98(2H, q)7.05(H, s) 7.60(H, s) | 2950, 1660, 1140, 1300, 1200 | glass-like |
| 23 | 23 | 1.55(3H, t)1.58(6H, s)2.15-2.50(2H, m) 2.42(3H, s)2.75(3H, s)3.20-3.50 (2H, m) 4.10(2H, q)7.05(H, s)7.35-7.60(2H, m) 7.70(H, s)7.75-7.90(H, m) | 2950, 3000, 1650, 1130, 1290, 1230 | 165.2-169.0 |
| 24 | 24 | 1.48(3H, t)1.60(6H, s)2.20(3H, s) 2.28-2.50(2H, m)2.40(3H, s)2.80(3H, s) 3.30-3.55(2H, m) 4.20(2H, q)5.40(2H, s) 7.00(H, s)7.15(H, s) | 2940, 2980, 1640, 1130, 1290, 1410 | glass-like |
| 25 | 25 | 1.50(6H, s)1. 60(3H, t) 2.20-2.50(2H, m) 2.35 (3H, s) 2.75(3H, s)3.25-3.50(2H, m) 4.30(2H, q)6.20(2H, s)6.95(H, s) 7.15(H, s)7.35-8.10(5H, m) | 2950, 1650, 1130, 1290, 1190 | 177.5-179.5 |

| | | | | |
|---|---|---|---|---|
| * Prep. Ex. No. = Preparation Example No. | | | | |
| ** Cpd. No. = Compound No. | | | | |
| *** Int.Std. = Internal Standard | | | | |

### Preparation Example 26

4,4,5,8-Tetramethyl-6-(1-methyl-5-hydroxypyrazol-4-yl)carbonylthiochroman-1,1-dioxide (Compound 26) in an amount of 1.7 g (yield 78 %) was obtained in the same manner as in Preparation Example 1 except that the 1-ethyl-5-hydroxypyrazole was replaced with 1-methyl-5-hydroxypyrazole.

### Preparation Example 27

0.80 Gram (2.2 mmol) of the 4,4,5,8-tetramethyl-6-(1-methyl-5-hydroxypyrazol-4-yl)-carbonylthiochroman-1,1-dioxi de (Compound 26) obtained in Preparation Example 26 was dissolved in 20 ml of methylene chloride. Then, a solution of 0.40 g (2.9 mmol) of potassium carbonate in 10 ml of water was added, and further, 0.43 g (3.8 mmol) of n-propanesulfonyl chloride and 0.05 g (0.2 mmol) of benzyl triethylammonium chloride were added. The mixture was allowed to react at room temperature for 24 hours. After the reaction, a methylene chloride layer was separated and recovered, and it was dried over anhydrous sodium sulfate. Then, the solvent was distilled off under reduced pressure, and the resultant oil was purified by silica gel chromatography, to give 0.60 g (yield 59 %) of 4,4,5,8-tetramethyl-6-(1- methyl -5-n-propanesulfonyloxypyrazol-4-yl)carbonylthiochroman-1,1-di oxide (Compound 27).

### Preparation Example 28

4,4,5,8-Tetramethyl-6-(1-methyl-5-p-toluenesulfonyloxypyrazol-4-yl)carbonylthiochroman-1,1-dioxide (Compound 28) in an amount of 0.68 g (yield 64 %) was obtained in the same manner as in Preparation Example 27 except that the n-propanesulfonyl chloride was replaced with p-toluenesulfonyl chloride.

### Preparation Example 29

5-Chloro-4,4,8-trimethyl-6-(1-ethyl-5-hydroxypyrazol-4-yl)carb onylthiochroman-1,1-dioxide in an amount of 0.52 g (yield 50 %) was obtained in the same manner as in Preparation Example 1 except that the 4,4,5,8-tetramethylthiochroman-6-carboxylic acid-1,1-dioxide was replaced with 5-chloro-4,4,8-trimethylthiochroman-6-carboxylic acid-1,1-dioxide.

Table 5 shows the structures and yields of Compounds 26 to 29 obtained in the above Preparation Examples 26 to 29 and their properties.

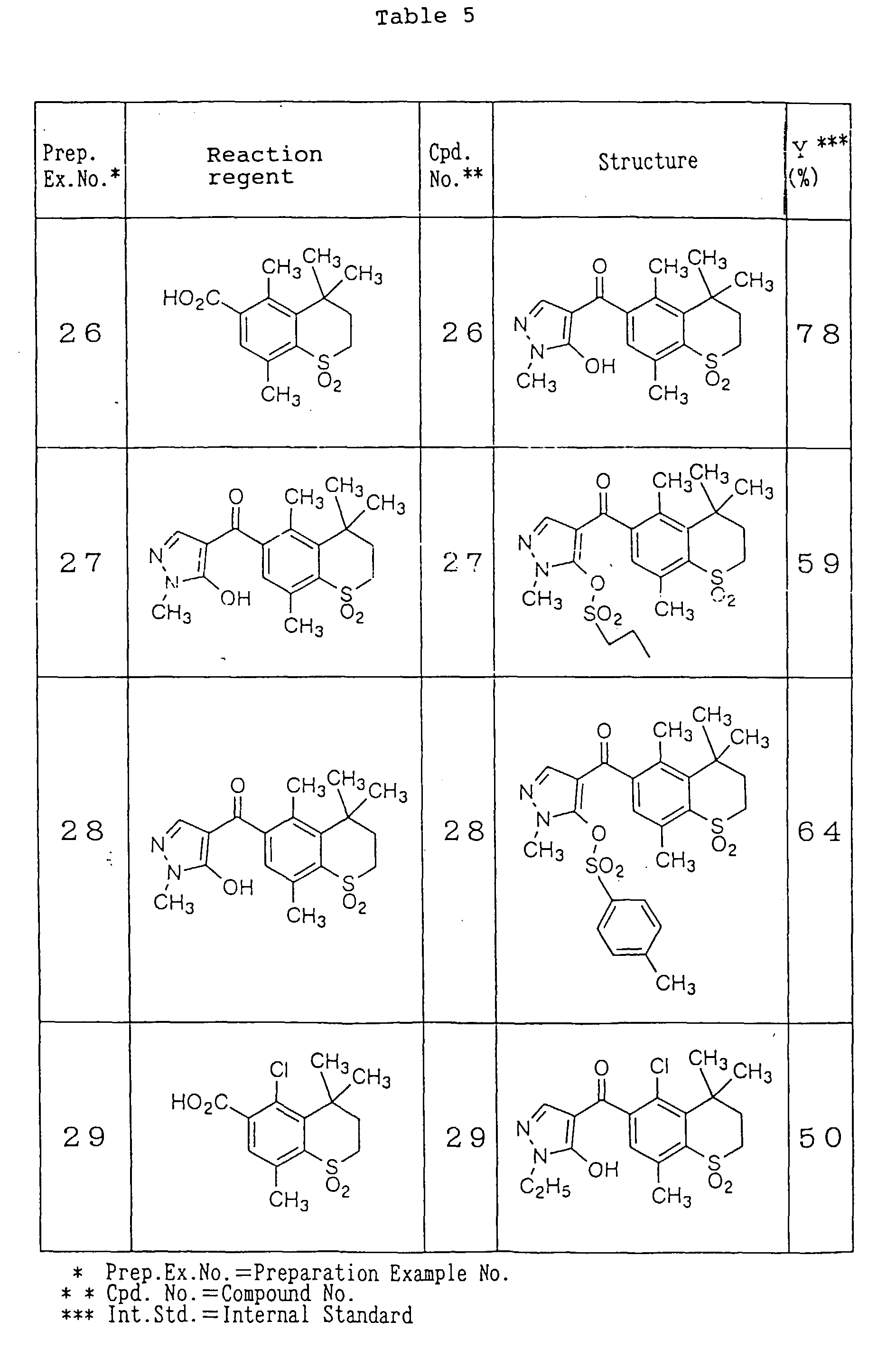

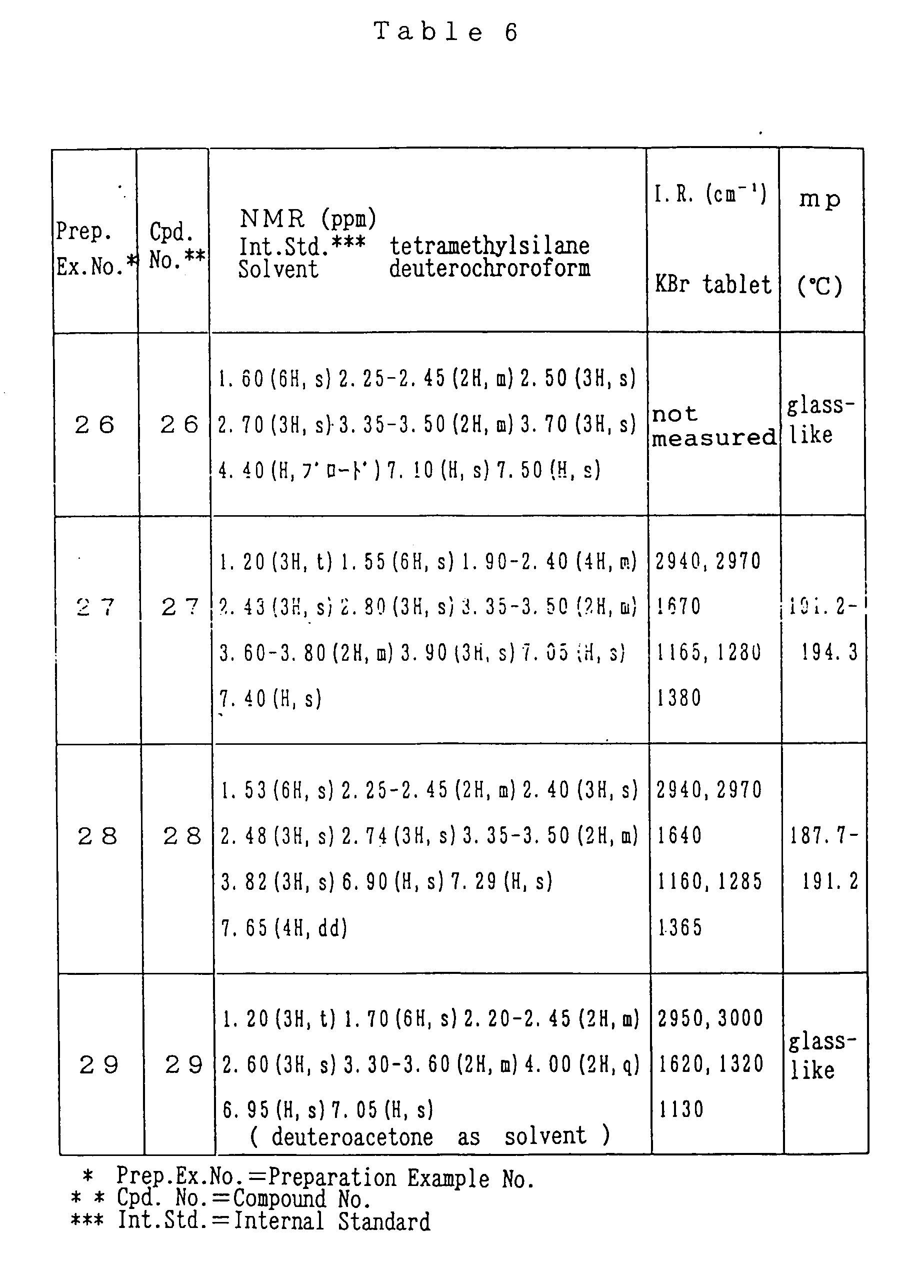

Examples of the herbicide to achieve the second object of the present invention will be described hereinafter.

### Herbicide Example

### (1) Preparation of Herbicide

97 Parts by weight of talc (trade name: Zeaklite) as a carrier, 1.5 parts by weight of alkylarylsulfonic acid (trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to obtain a carrier for a wettable powder.

90 Parts by weight of the above carrier and 10 parts by weight of one compound taken from Compounds of the present invention obtained in the above Preparation Examples were uniformly pulverized and mixed to obtain a herbicide. Comparative herbicides were prepared from the following compounds x, y, A, B and C in an amount of 10 parts by weight each in the same manner as above.

### Compound (x): Commercially available herbicide pyrazolate

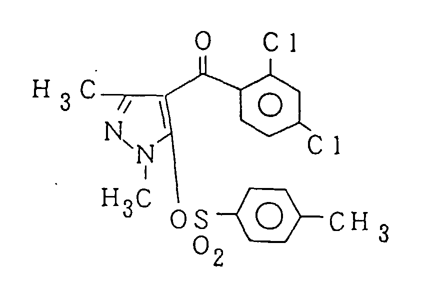

### Compound (y): Compound described in JP-A-63-122672

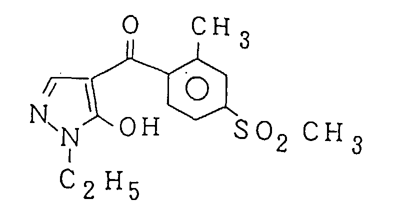

### Compound (A): Compound No. 35 described in JP-A-2-173

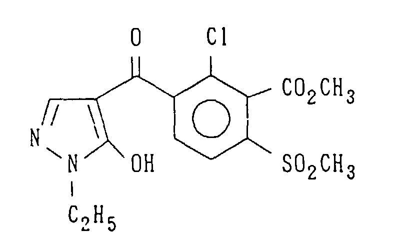

### Compound (B): Compound No. 66 described in International Publication No. WO93/18031

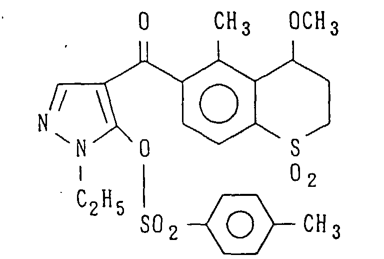

### Compound (C): Compound No. b-3 described in Japanese Patent Application No. 4-185526

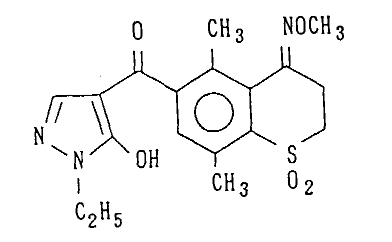

### (2) Biological test (Foliar treatment test,

### Herbicide Examples 1 and 2, and Comparative Examples 1 and 2)

Seeds of weeds such as large crabgrass, barnyardgrass, green foxtail, cocklebur, velvetleaf and slender amaranth and seeds of corn, wheat and barley were sown in 1/5,000 are Wagner pots filled with upland soil, and covered with upland soil. Then, the seeds were grown in a greenhouse, and when they grew to plants at one or two-leaved stage, a predetermined amount of the herbicide prepared in the above (1) was suspended in water and uniformly sprayed to foliar portions at a rate of 200 liter/10 are. Thereafter, the plants were grown in the greenhouse, and 20 days after the spraying treatment, the herbicide was determined for herbicidal efficacy. Table 7 shows the results.

The herbicidal efficacy and the phytotoxicity to the crops are shown on the basis of the following ratings.

The remaining plant weight/non-treated ratio was calculated by (remaining plant weight in treated plot/remaining plant weight in non-treated plot) x 100.

| (Ratings) Herbicidal efficacy | Remaining plant weight/non-treated ratio (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity | Remaining plant weight/non-treated ratio (%) |
|---|---|
| - | 100 |
| ± | 95 - 99 |
| + | 90 - 94 |
| ++ | 80 - 89 |
| +++ | 0 - 79 |

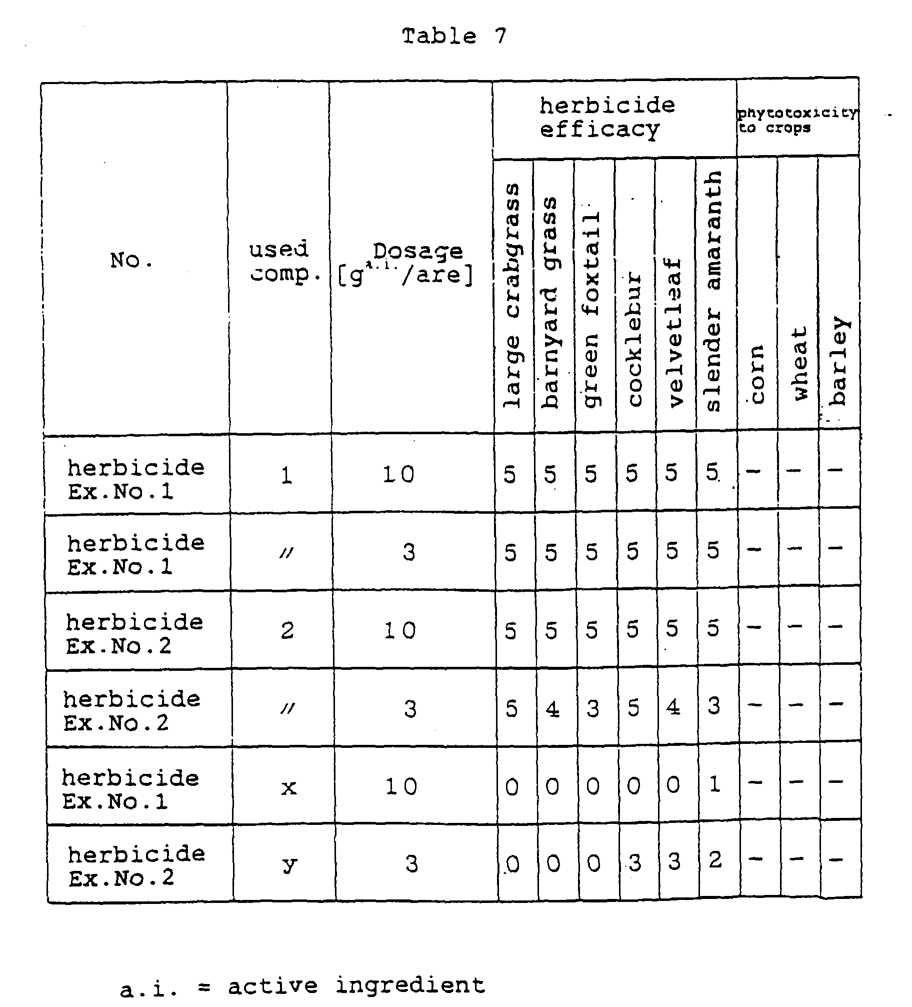

### (3) Biological test (Upland soil treatment test,

### Herbicide Examples 3 and 4, and Herbicide Comparative Examples 3 and 4)

Seeds of weeds such as large crabgrass, barnyardgrass, green foxtail, cocklebur, velvetleaf and slender amaranth and seeds of corn, wheat and barley were sown in 1/5,000 are Wagner pots filled with upland soil, and covered with upland soil. Then, a predetermined amount of the herbicide prepared in the above (1) was suspended in water and uniformly sprayed onto the soil surface. Thereafter the seeds were grown in the greenhouse, and 20 days after the spraying treatment, the herbicide was determined for herbicidal efficacy. Table 8 shows the results.

The herbicidal efficacy and the phytotoxicity to the crops were expressed according to the ratings specified in the (2) Foliar treatment test.

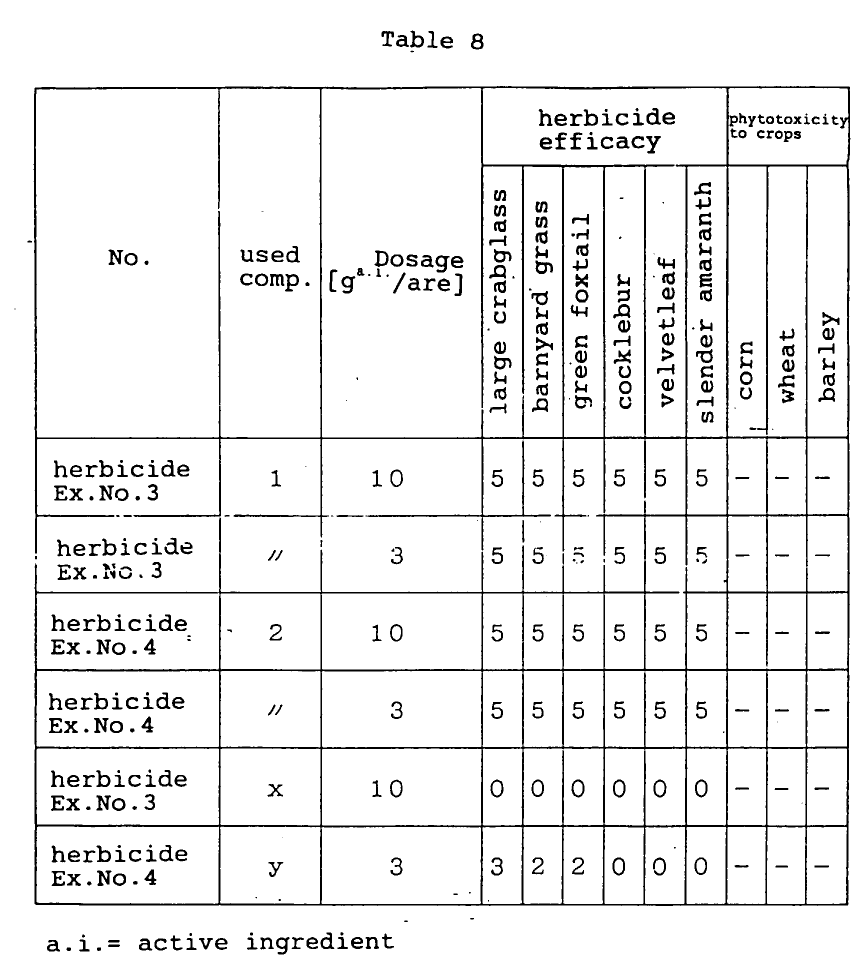

### (4) Biological test (Submergence soil treatment test, Herbicide Examples 5 - 9, Comparative Examples 5 - 8, and Referential Examples 1 and 2)

A 1/15,500-are porcelain pot was filled with paddy field soil, and seeds of barnyardgrass and umbrella plant were uniformly sown in a surface layer of the soil, and paddy rice at the two-leaf stage was transplanted. Thereafter, a diluted solution of a predetermined amount of the herbicide prepared in the above (1) was uniformly sprayed onto the water surface at the time of germination of the weeds, and then the pot was allowed to stand in a greenhouse while water was properly sprayed. Twenty days after the treatment with the herbicide solution, the herbicidal efficacy and phytotoxicity to paddy rice were inspected, and Table 9 shows the results. The dosage of the herbicide is shown as an amount of the effective ingredient per 10 ares. Further, air-dried weights were measured, and the phytotoxicity to paddy rice and the herbicidal efficacy were shown as follows.

| Herbicidal efficacy | Ratio of remaining plant weight to non-treated (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to paddy rice | Ratio of remaining plant weight to non-treated (%) |
|---|---|
| 0 | 100 |
| 1 | 95 - 99 |
| 2 | 90 - 94 |
| 3 | 80 - 89 |
| 4 | 0 - 79 |

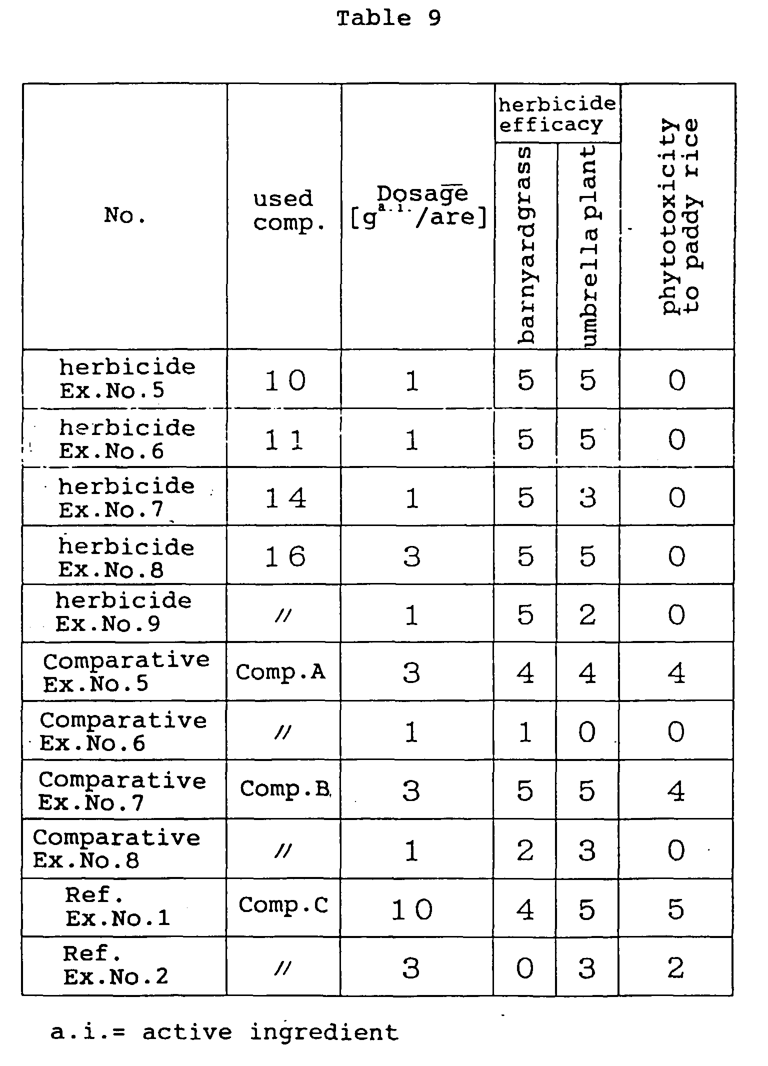

### (5) Biological test (Soil treatment test on upland soil, Herbicide Examples 10 - 26 and

### Comparative Example 9)

Seeds of weeds such as large crabgrass, barnyardgrass, green foxtail, cocklebur, velvetleaf and slender amaranth and seeds of corn were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the seeds were grown in a greenhouse, and 20 days after the treatment, the herbicide was determined for herbicidal efficacy and phytotoxicity to corn. Table 10 shows the results.

The dosage of the herbicide is shown as an amount of the effective ingredient per hectare. Further, air-dried weights were measured, and herbicidal efficacy and the phytotoxicity to corn were shown as follows.

| Herbicidal efficacy | Ratio of remaining plant weight to non-treated (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to corn | Ratio of remaining plant weight to non-treated (%) |
|---|---|
| 0 | 100 |
| 1 | 95 - 99 |
| 2 | 90 - 94 |
| 3 | 80 - 89 |
| 4 | 0 - 79 |

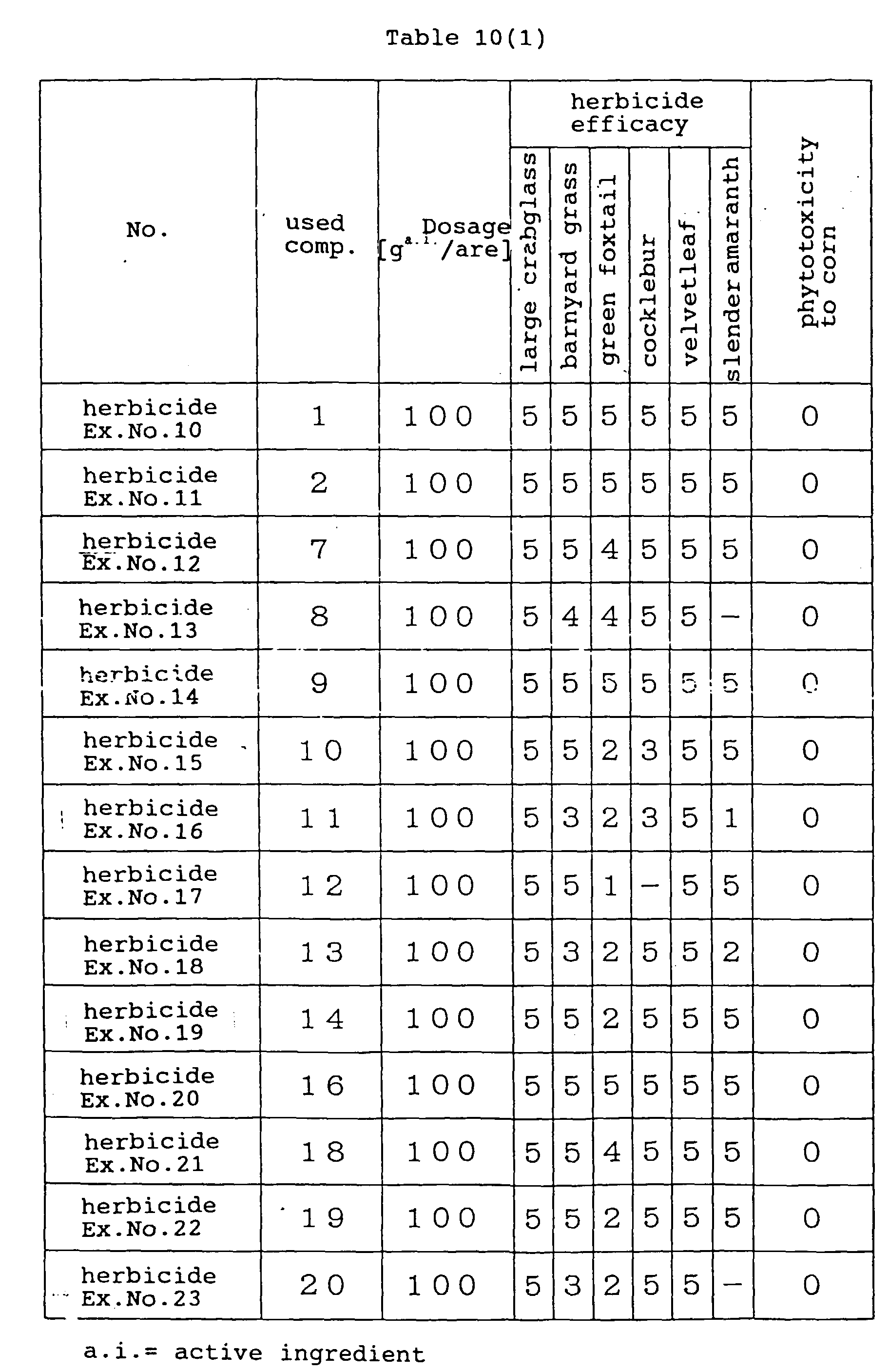

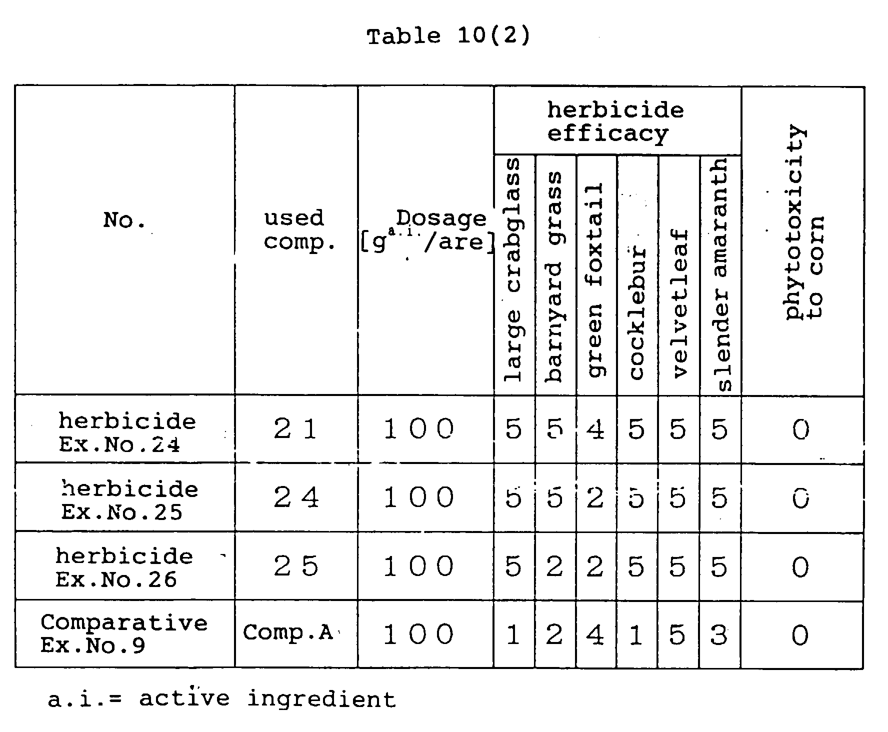

### Effect of the Invention

As specified above, the present invention provides pyrazole derivatives which can control gramineous weeds and broad-leaved weeds together at a low dosage by any treatment of foliar treatment and soil treatment without causing phytotoxicity to rice, corn, wheat and barley, herbicides containing the above pyrazole derivatives as active ingredient, and intermediate compounds suitable for the production of the above pyrazole derivatives.

## Claims

1. A pyrazole derivative of the formula (I), 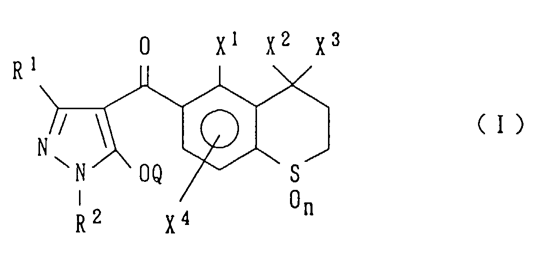 {wherein:
R¹ is a hydrogen atom or a C₁-C₄ alkyl group;
R² is a C₁-C₄ alkyl group;
X¹ is a hydrogen atom, a halogen atom or a C₁-C₄ alkyl group;
each of X² and X³ is independently a C₁-C₄ alkyl group;
X⁴ is a hydrogen atom, or a halogen atom or C₁-C₄ alkyl group substituted on the 7-position or 8-position of a thiochroman ring;
Q is a hydrogen atom or a group of the formula of -A-B [in which:
A is -SO₂-, -CO- or -CH₂CO-; and
B is a C₁-C₈ alkyl group, a C₃-C₈ cycloalkyl group or a group of the formula (V), 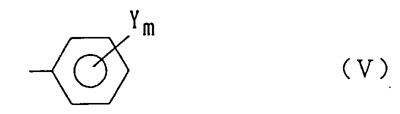 (in which:
Y is a halogen atom, a nitro group, a C₁-C₄ alkyl group, a C₁-C₄ alkoxy group or a halo C₁-C₄ alkyl group; and
m is an integer of 0, 1 or 2)]; and
n is an integer of 0, 1 or 2}, or a salt thereof.

2. The pyrazole derivative or its salt as recited in Claim 1, wherein R¹ is a hydrogen atom.

3. The pyrazole derivative or its salt as recited in Claim 1, wherein R² is methyl or ethyl.

4. The pyrazole derivative or its salt as recited in Claim 1, wherein X¹ is a hydrogen atom, methyl or a chlorine atom.

5. The pyrazole derivative or its salt as recited in Claim 1, wherein each of X² and X³ is methyl.

6. The pyrazole derivative or its salt as recited in Claim 1, wherein X⁴ is a hydrogen atom, or methyl or a chlorine atom substituted on the 8-position of a thiochroman ring.

7. The pyrazole derivative or its salt as recited in Claim 1, wherein Q is a hydrogen atom.

8. The pyrazole derivative or its salt as recited in Claim 1, wherein Q is the group of the formula of -A-B in which A is -SO₂- and B is a C₁-C₈ alkyl group or the group of the formula (V), 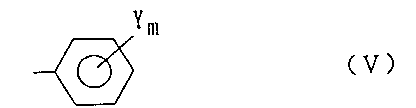 (in which Y is a halogen atom, a nitro group, a C₁-C₄ alkyl group or a C₁-C₄ alkoxy group and m is 1 or 2).

9. The pyrazole derivative or its salt as recited in Claim 1, wherein Q is the group of the formula of -A-B in which A is -CO- or -CH₂CO- and B is a C₁-C₈ alkyl group, a C₃-C₈ cycloalkyl group or the group of the formula (V), 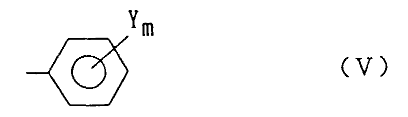 (in which Y is a halogen atom and m is 0, 1 or 2).

10. A herbicide containing, as an active ingredient, the pyrazole derivative and/or its salt as recited in any one of Claims 1 to 9.

11. An aromatic carboxylic acid derivative of the formula (II), 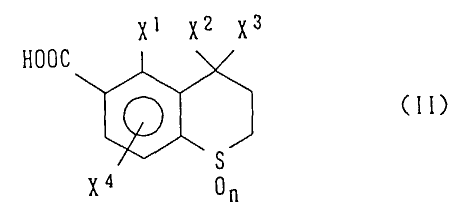 (wherein:
X¹ is a C₁-C₄ alkyl group or a halogen atom;
each of X² and X³ is independently a C₁-C₄ alkyl group; and
X⁴ is a hydrogen atom, or a C₁-C₄ alkyl group or halogen atom substituted on the 7-position or 8-position of a thiochroman ring),
or a salt thereof.

## Patentansprüche

1. Pyrazol-Derivat der Formel (I) 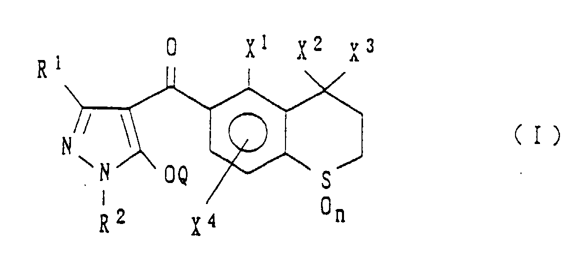 {wobei:
R¹ ein Wasserstoffatom oder eine (C₁-C₄)Alkylgruppe ist;
R² eine (C₁-C₄)Alkylgruppe ist;
X¹ ein Wasserstoffatom, ein Halogenatom oder eine (C₁-C₄)Alkylgruppe ist;
jedes von X² und X³ unabhängig voneinander eine (C₁-C₄)Alkylgruppe ist;
X⁴ ein Wasserstoffatom oder ein Halogenatom oder eine (C₁-C₄)Alkylgruppe ist, die an der 7-Position oder 8-Position eines Thiochromanrings substituiert ist;
Q ein Wasserstoffatom oder eine Gruppe der Formel -A-B ist [wobei:
A - SO₂-, -CO- oder -CH₂CO- ist; und
B eine (C₁-C₈)Alkylgruppe, eine (C₃-C₈)Cycloalkylgruppe oder eine Gruppe der Formel (V) ist 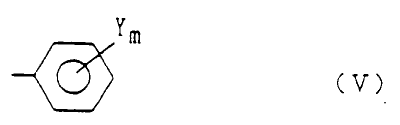 (wobei:
Y ein Halogenatom, eine Nitrogruppe, eine (C₁-C₄)Alkylgruppe, eine (C₁-C₄)Alkoxygruppe oder eine Halo(C₁-C₄)alkylgruppe ist; und
m eine ganze Zahl von 0, 1 oder 2 ist)]; und
n eine ganze Zahl von 0, 1 oder 2 ist}, oder ein Salz davon.

2. Pyrazol-Derivat oder sein Salz wie in Anspruch 1 angegeben, wobei R¹ ein Wasserstoffatom ist.

3. Pyrazol-Derivat oder Salz davon wie in Anspruch 1 angegeben, wobei R² Methyl oder Ethyl ist.

4. Pyrazol-Derivat oder Salz davon wie in Anspruch 1 angegeben, wobei X¹ ein Wasserstoffatom, Methyl oder ein Chloratom ist.

5. Pyrazol-Derivat oder sein Salz davon wie in Anspruch 1 angegeben, wobei jeder von X² und X³ Methyl ist.

6. Pyrazol-Derivat oder Salz davon wie in Anspruch 1 angegeben, wobei X⁴ ein Wasserstoffatom oder Methyl oder ein Chloratom ist, das an der 8-Position eines Thiochromanrings substituiert ist.

7. Pyrazol-Derivat oder sein Salz wie in Anspruch 1 angegeben, wobei Q ein Wasserstoffatom ist.

8. Pyrazol-Derivat oder sein Salz wie in Anspruch 1 angegeben, wobei Q die Gruppe der Formel - A-B ist, wobei A -SO₂- ist und B eine (C₁-C₈)Alkylgruppe oder die Gruppe der Formel (V) ist 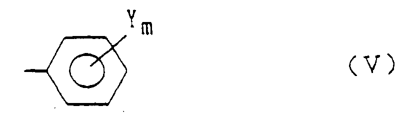 (wobei: Y ein Halogenatom, eine Nitrogruppe, eine (C₁-C₄)Alkylgruppe oder eine (C₁-C₄)Alkoxygruppe und m 1 oder 2 ist).

9. Pyrazol-Derivat oder sein Salz wie in Anspruch 1 angegeben, wobei Q die Gruppe der Formel -A-B ist, wobei A - CO- oder -CH₂CO- ist und B eine (C₁-C₈)Alkylgruppe, eine (C₃-C₈)Cycloalkylgruppe oder die Gruppe der Formel (V) ist 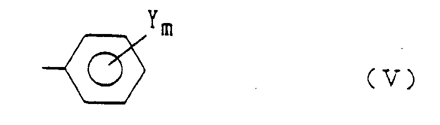 (wobei Y ein Halogenatom ist und m 0, 1 oder 2 ist).

10. Herbizid, enthaltend als aktiven Bestandteil das Pyrazol-Derivat und/oder sein Salz wie in einem der Ansprüche 1 bis 9 angegeben.

11. Aromatisches Carbonsäure-Derivat der Formel (II) 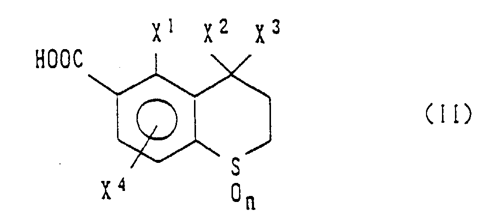 (wobei:
X¹ eine (C₁-C₄)Alkylgruppe oder ein Halogenatom ist; jedes von X² und X³ unabhängig voneinander eine (C₁-C₄)Alkylgruppe ist; und X⁴ ein Wasserstoffatom oder eine (C₁-C₄)Alkylgruppe oder ein Halogenatom ist, das an der 7-Position oder 8-Position eines Thiochromanrings substituiert ist),
oder ein Salz davon.

## Revendications

1. Dérivé de pyrazole de formule (I) 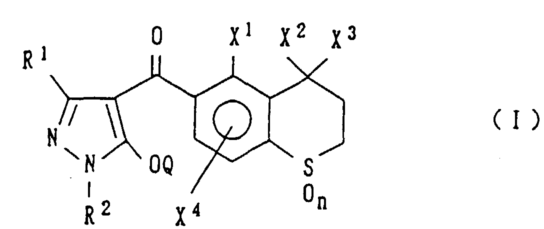 {dans laquelle:
R¹ est un atome d'hydrogène ou un groupe alkyle en C₁-C₄;
R² est un groupe alkyle en C₁-C₄;
X¹ est un atome d'hydrogène, un atome d'halogène ou un groupe alkyle en C₁-C₄;
chacun des radicaux X² et X³ est indépendamment un groupe alkyle en C₁-C₄;
X⁴ est un atome d'hydrogène, ou un atome d'halogène ou un groupe alkyle en C₁-C₄ substitué en position 7 ou 8 d'un noyau thiochromane; Q est un atome d'hydrogène ou un groupe de formule -A-B
[dans laquelle:
A est -SO₂-, -CO- ou -CH₂CO-; et
B est un groupe alkyle en C₁-C₈, un groupe cycloalkyle en C₃-C₈ ou un groupe de formule (V), 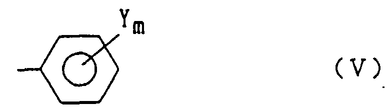 (dans laquelle:
Y est un atome d'halogène, un groupe nitro, un groupe alkyle en C₁-C₄, un groupe alcoxy en C₁-C₄ ou un groupe halogénoalkyle en C₁-C₄; et
m est un nombre entier égal à 0, 1 ou 2)]; et
n est un nombre entier égal à 0, 1 ou 2},
ou un de ses sels.

2. Dérivé de pyrazole ou son sel selon la revendication 1, dans lequel R' est un atome d'hydrogène.

3. Dérivé de pyrazole ou son sel selon la revendication 1, dans lequel R² est un groupe méthyle ou éthyle.

4. Dérivé de pyrazole ou son sel selon la revendication 1, dans lequel X¹ est un atome d'hydrogène, un groupe méthyle ou un atome de chlore.

5. Dérivé de pyrazole ou son sel selon la revendication 1, dans lequel chacun des radicaux X² et X³ est un groupe méthyle.

6. Dérivé de pyrazole ou son sel selon la revendication 1, dans lequel X⁴ est un atome d'hydrogène ou un groupe méthyle ou un atome de chlore substitué en position 8 d'un noyau thiochromane.

7. Dérivé de pyrazole ou son sel selon la revendication 1, dans lequel Q est un atome d'hydrogène.

8. Dérivé de pyrazole ou son sel selon la revendication 1, dans lequel Q est le groupe de formule -A-B dans laquelle A est -SO₂- et B est un groupe alkyle en C₁-C₈ ou le groupe de formule (V), 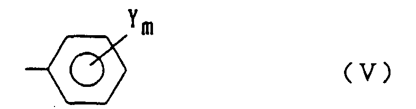 (dans laquelle Y est un atome d'halogène, un groupe nitro, un groupe alkyle en C₁-C₄ ou un groupe alcoxy en C₁-C₄ et m vaut 1 ou 2).

9. Dérivé de pyrazole ou son sel selon la revendication 1, dans lequel Q est le groupe de formule -A-B dans laquelle A est -CO- ou -CH₂CO- et B est un groupe alkyle en C₁-C₈, un groupe cycloalkyle en C₃-C₈ ou le groupe de formule (V), 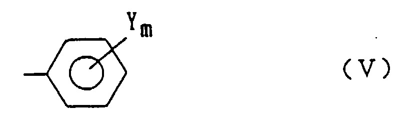 (dans laquelle Y est un atome d'halogène et m vaut 0, 1 ou 2).

10. Herbicide contenant, comme ingrédient actif, le dérivé de pyrazole et/ou son sel selon l'une quelconque des revendications 1 à 9.

11. Dérivé d'acide carboxylique aromatique de formule (II) 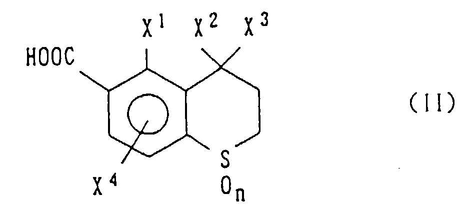 (dans laquelle:
X¹ est un groupe alkyle en C₁-C₄ ou un atome d'halogène;
chacun des radicaux X² et X³ est indépendamment un groupe alkyle en C₁-C₄;
et
X⁴ est un atome d'hydrogène, ou un groupe alkyle en C₁-C₄ ou un atome d'halogène substitué en position 7 ou en position 8 d'un noyau thiochromane),
ou un de ses sels.
